# EUROPÄISCHE PATENTSCHRIFT

(11) **EP 1 345 932 B1**
(45) Veröffentlichungstag und Bekanntmachung des Hinweises auf die Patenterteilung: **18.10.2006**
(21) Anmeldenummer: 01271371.5
(22) Anmeldetag: 11.12.2001
(51) Int. Cl.: C07D 409/12, A61K 31/397, A61P 9/00

(54) **DIPHENYLAZETIDINONDERIVATE, VERFAHREN ZU DEREN HERSTELLUNG, DIESE VERBINDUNGEN ENTHALTENDE ARZNEIMITTEL UND DEREN VERWENDUNG**
DIPHENYL AZETIDINONE DERIVATIVES, METHOD FOR THE PRODUCTION THEREOF, MEDICAMENTS CONTAINING THESE COMPOUNDS, AND THEIR USE
DERIVES DE DIPHENYLAZETIDINONE, PROCEDE PERMETTANT DE LES PRODUIRE, MEDICAMENTS CONTENANT LESDITS COMPOSES ET LEUR UTILISATION

(30) Priorität: 21.12.2000 DE 10064402; 07.11.2001 DE 10154520
(43) Veröffentlichungstag der Anmeldung: 24.09.2003
(73) Patentinhaber: Sanofi-Aventis Deutschland GmbH, 65929 Frankfurt am Main (DE)
(72) Erfinder: GLOMBIK, Heiner, 65719 Hofheim (DE); KRAMER, Werner, 55130 Mainz-Laubenheim (DE); FLOHR, Stefanie, CH-4054 Basel (DE); FRICK, Wendelin, 65510 Hünstetten-Beuerbach (DE); HEUER, Hubert, 55270 Schwabenheim (DE); JAEHNE, Gerhard, 65929 Frankfurt (DE); LINDENSCHMIDT, Andreas, 65812 Bad Soden (DE); SCHAEFER, Hans-Ludwig, 65239 Hochheim (DE)
(86) Internationale Anmeldenummer: PCT/EP2001/014532
(87) Internationale Veröffentlichungsnummer: WO 2002/050068

(56) Entgegenhaltungen:
- WO-A-00/01687
- WO-A-95/08532
- WO-A-95/35277
- WO-A-96/08484
- WO-A-96/19450

## Beschreibung

Die Erfindung betrifft substituierte Diphenylazetidinone, deren physiologisch verträgliche Salze sowie physiologisch funktionelle Derivate.

Es sind bereits Diphenylazetidinone (wie z.B. Ezetimibe) sowie deren Verwendung zur Behandlung von Hyperlipidämie sowie Arteriosklerose und Hypercholesterinämie beschrieben worden [vgl. Drugs of the Future 2000, 25(7):679-685].

Der Erfindung lag die Aufgabe zugrunde, weitere Verbindungen zur Verfügung zu stellen, die eine therapeutisch verwertbare hypolipidämische Wirkung entfalten. Insbesonders bestand die Aufgabe darin, neue Verbindungen zu finden, die gegenüber den im Stand der Technik beschriebenen Verbindungen sehr gering resorbierbar sind. Unter sehr gering resorbierbar wird eine intestinale Resorption kleiner 10%, bevorzugt kleiner oder gleich 5% verstanden.
Die neuen Verbindungen sollen insbesonders eine geringere Resorption als Ezetimibe auf weisen.
Bei geringerer Resorption zeigen pharmazeutische Wirkstoffe in der Regel deutlich weniger Nebenwirkungen.

Die Erfindung betrifft daher Verbindungen der Formel I, 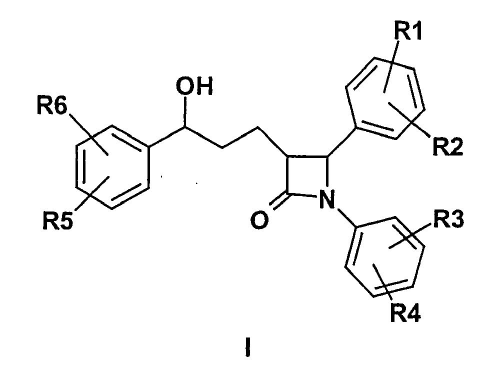 worin bedeuten
- R1, R2, R3, R4, R5, R6: unabhängig voneinander (C₀-C₃₀)-Alkylen-L, wobei ein oder mehrere C-Atome des Alkylenrests durch -O-, -(C=O)-, -CH=CH, -C≡C-, -N((C₁-C₆)Alkyl)- oder -NH- ersetzt sein können;
H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- L: 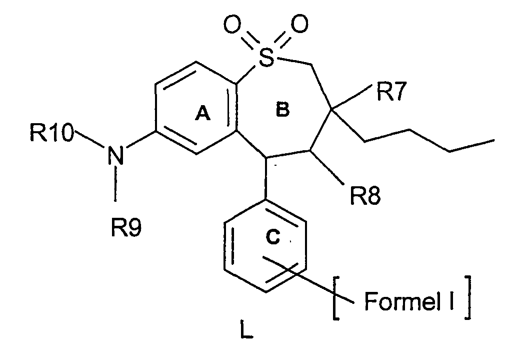
- R7: Methyl, Ethyl, Propyl, Butyl;
- R8: H, OH, NH₂, NH-(C₁-C₆)-Alkyl;
- R9: Methyl, Ethyl, Propyl, Butyl;
- R10: Methyl, Ethyl, Propyl, Butyl;
wobei immer mindestens einer der Reste R1 bis R6 die Bedeutung (C₀-C₃₀)-Alkylen-L, wobei ein oder mehrere C-Atome des Alkylenrests durch -O-, -(C=O)-, -CH=CH-, -C≡C-, -N((C₁-C₆)-Alkyl)- oder -NH- ersetzt sein können, besitzen muß,
sowie deren pharmazeutisch verträglichen Salze.

Bevorzugt sind Verbindungen der Formel I, worin mindestens einer der Reste R1 bis R6 die Bedeutung (C₀-C₃₀)-Alkylen-L, wobei ein oder mehrere C-Atome des Alkylenrests durch -O-, -(C=O)- oder -NH- ersetzt sein können, besitzt.

Besonders bevorzugt sind Verbindungen der Formel I, worin einer der Reste R1 oder R3 die Bedeutung (C₀-C₃₀)-Alkylen-L hat, wobei ein oder mehrere C-Atome des Alkylenrests durch -O-, -(C=O)- oder -NH- ersetzt sein können.

Ganz besonders bevorzugt sind Verbindungen der Formel I, worin einer der Reste R1 oder R3 die Bedeutung -(CH₂)₀₋₁-NH-(C=O)₀₋₁-(C₃-C₂₅)-Alkylen-(C=O)₀₋₁-NH-L hat, wobei ein oder mehrere C-Atome des Alkylenrests durch O-Atome ersetzt sein können.

Die Verknüpfung eines der Reste R1 bis R6 mit dem L-Rest erfolgt bevorzugt in Meta-Stellung des Rings C der L-Gruppe.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chloridsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung, z.B. ein Ester, das bei Verabreichung an einen Säuger, wie z.B. den Menschen, in der Lage ist, (direkt oder indirekt) eine solche Verbindung oder einen aktiven Metaboliten hiervon zu bilden.

Ein weiterer Aspekt dieser Erfindung sind Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Verbindungen der Formel I und deren pharmazeutisch verträgliche Salze und physiologisch funktionelle Derivate stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen.

Die Verbindung(en) der Formel (I) können auch in Kombination mit weiteren Wirkstoffen verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Diphenylazetidinon-Ions. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

### Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesonders zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Antidiabetika umfassen Insulin und Insulinderivate, wie z.B. Lantus® oder HMR 1964, GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguadine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlididämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazone, Pioglitazone, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, verabreicht.

Bei einer Ausführungsform der Erfindung werden die Verbindungen der Formel I in Kombination mit einem Fibrat, wie z.B. Fenofibrate, Clofibrate, Bezafibrate, verabreicht.

Bei einer Ausführungsform der Erfindung werden die Verbindungen der Formel I in Kombination mit einem MTP-Inhibitor, wie z.B. Bay 13-9952, BMS-201038, R-103757, verabreicht.

Bei einer Ausführungsform der Erfindung werden die Verbindungen der Formel I in Kombination mit Gallensäureresorptionsinhibitor, wie z.B. HMR 1453, verabreicht.

Bei einer Ausführungsform der Erfindung werden die Verbindungen der Formel I in Kombination mit einem CETP-Inhibitor, wie z.B. Bay 194789, verabreicht.

Bei einer Ausführungsform der Erfindung werden die Verbindungen der Formel I in Kombination mit einem polymeren Gallensäureadsorber, wie z.B. Cholestyramine, Colesolvam, verabreicht.

Bei einer Ausführungsform der Erfindung werden die Verbindungen der Formel I in Kombination mit einem LDL-Rezeptorinducer, wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalene synthetase inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamide, Glibenclamide, Glipizide oder Gliclazide, verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinide, verabreicht.

Bei einer Ausführungsform werden die Verbindungen der Formel I in Kombination mit einem Thiazolidindion, wie z.B. Troglitazone, Ciglitazone, Pioglitazone, Rosiglitazone oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamide, Glibenclamide, Glipizide, Gliazide oder Repaglinide.

Bei einer Ausführungsform werden die Verbindungen der Formel 1 in Kombination mit mehr als einer der vorstehend genannten Verbindungen, z.B. in Kombination mit einem Sulphonylharnstoff und Metformin, einem Sulphonylharnstoff und Acarbose, Repaglinide und Metformin, Insulin und einem Sulphonylharnstoff, Insulin und Metformin, Insulin und Troglitazone, Insulin und Lovastatin, etc. verabreicht.

Bei einer weiteren Ausführungsform werden die Verbindungen der Formel I in Kombination mit CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyte-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormon, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamine oder Amphetamin.

Bei einer Ausführungsform ist der weitere Wirkstoff Fenfluramine oder Dexfenfluramin.

Bei noch einer Ausführungsform ist der weitere Wirkstoff Sibutramine.

Bei einer Ausführungsform ist der weitere Wirkstoff Orlistat.

Bei einer Ausführungsform ist der weitere Wirkstoff Mazindol oder Phentermine.

Bei einer Ausführungsform werden die Verbindungen der Formel I in Kombination mit Ballaststoffen, vorzugsweise unlöslichen Ballaststoffen, wie z.B. Caromax® verabreicht. Die Kombination mit Caromax® kann in einer Zubereitung erfolgen, oder durch getrennte Gabe von Verbindungen der Formel I und Caromax®. Caromax® kann dabei auch in Form von Lebensmitteln, wie z.B. in Backwaren oder Müsliriegeln, verabreicht werden. Die Kombination von Verbindungen der Formel I mit Caromax® zeichnet sich neben einer Wirkverbesserung, insbesonders in der LDL-Cholesterinsenkung, gegenüber den Einzelwirkstoffen, auch durch Ihre verbesserte Verträglichkeit aus.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Bevorzugt sind racemische als auch enantiomerenreine Verbindungen der Formel I mit folgender Struktur:

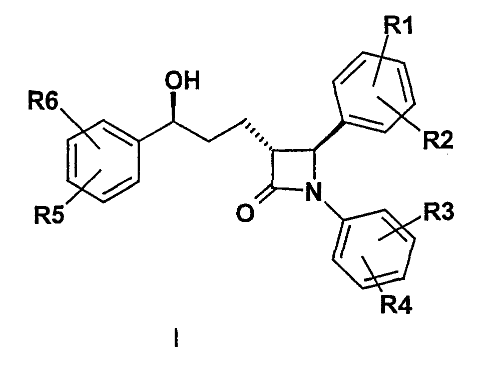

Weiterhin bevorzugt sind Verbindungen der Formel I, worin die L Reste folgende Bedeutung haben:

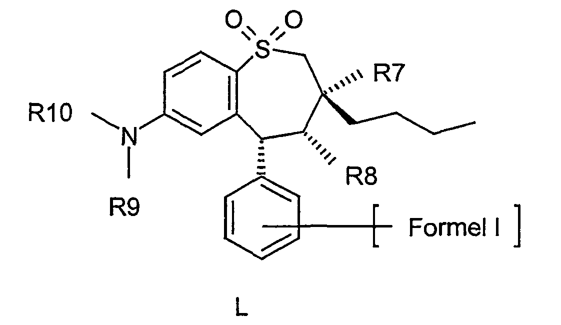

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß analog dem folgenden Reaktionsschema vorgegangen wird.

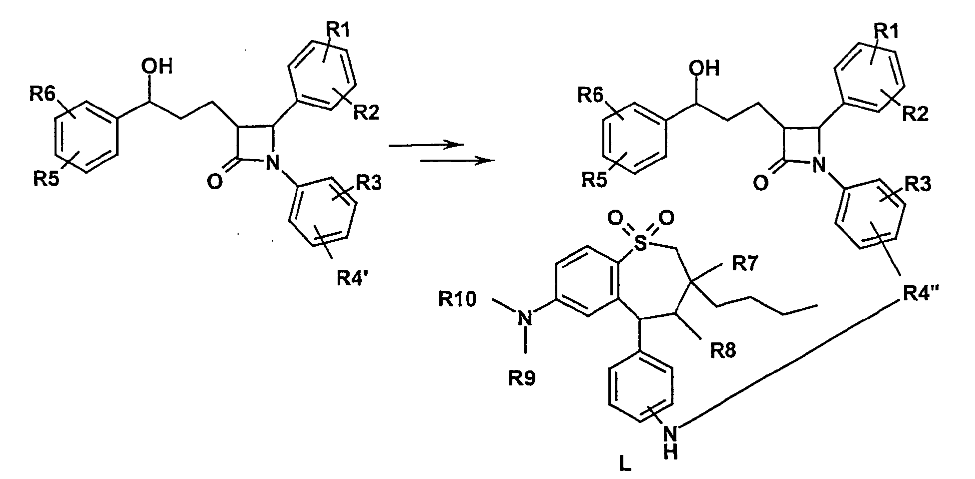

R4" bedeutet (C₀-C₃₀)-Alkylen, wobei ein oder mehrere C-Atome des Alkylenrests, durch -O-, -(C=O)-, -CH=CH-, -C≡C-, -N((C₁-C₆)-Alkyl)- oder -NH- ersetzt sein können.

Alternativ erfolgt die Verknüpfung zur L-Gruppe über Ring A oder Ring C.

Die nachfolgenden Beispiele dienen zur näheren Erläuterung der Erfindung, ohne dieselbe auf in den Beispielen beschriebene Produkte und Ausführungsformen einzuschränken.

### Beispiel I

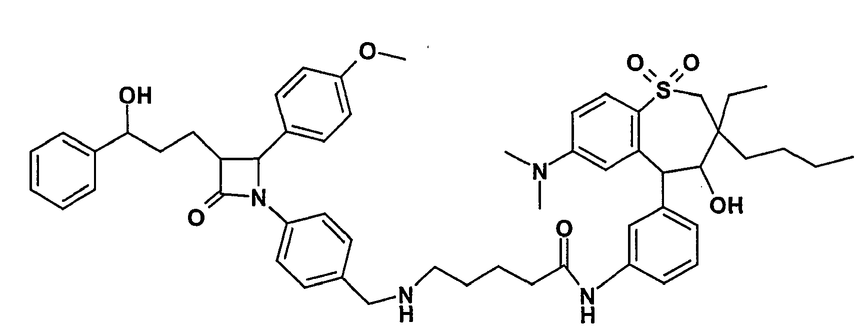

### 5-{4-[3-(3-Hydroxy-3-phenyl-propyl)-2-(4-methoxy-phenyl)-4-oxo-azetidin-1-yl]-benzylamino}-pentansäure-[3-(3-butyl-7-dimethylamino-3-ethyl-4-hydroxy-1,1-dioxo-2,3,4,5-tetrahydro-1H-benzo[b]thiepin-5-yl)-phenyl]-amid (1)

100 mg 5-Brom-pentansäure- [3-(3-butyl-7-dimethylamino-3-ethyl-4-hydroxy-1,1-dioxo-2,3,4,5- tetrahydro-1H-benzo[b]thiepin-5-yl)-phenyl]-amid und 70 mg 1-(4-Aminomethyl-phenyl)3-(3-hydroxy-3-phenyl-propyl)-4-(4-methoxy-phenyl)-azetidin-2- on werden in 5 ml Dimethylformamid gelöst und ca. 2 bis 3 Std. unter Rühren auf 80° C erwärmt. Nach beendeter Reaktion (Kontrolle durch Dünnschichtchromatogramm oder HPLC-MS) wird das Lösemittel im Vakuum entfernt und der Rückstand durch Chromatographie gereinigt. Man erhält so das Produkt 1 mit dem Molekulargewicht 929.24 (C₅₅H₆₈N₄O₇S); MS (FAB): 929 (M+H⁺).

### Beispiel II

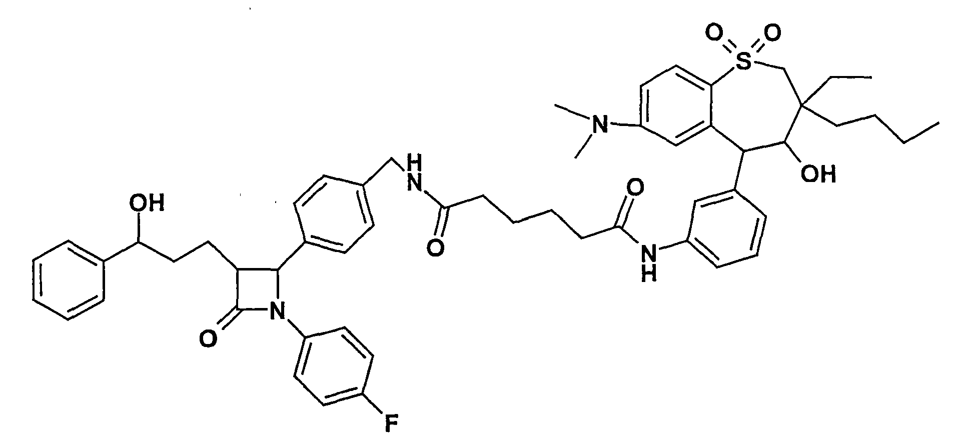

### Hexandisäure [3-(3-butyl-7-dimethylamino-3-ethyl-4-hydroxy-1,1-dioxo-2,3,4,5-tetrahydro-1H-benzo[b]thiepin-5-yl)-phenyl]-amid 4-[1-(4-fluor-phenyl)-3-(3- hydroxy-3-phenyl-propyl)-4-oxo-azetidin-2-yl]-benzylamid (8)

### a) 1-(2-Oxo-4-phenyl-oxazolidin-3-yl)-5-phenyl-pentan-1,5-dion (2)

10 g Benzoylbuttersäure und 12.5 ml Triethylamin werden in 55 ml Dichlormethan gelöst. Nach 5 min bei Raumtemp. gibt man 6.2 ml Pivaloylchlorid innerhalb von 30 min dazu und rührt 2 Std. Dann werden 5.9 g 4-Phenyl-oxazolidin-2-on in 6 ml Dimethylformamid und 0.9 g 4-(Dimethylamino)-pyridin zugesetzt. Man erwärmt ca. 7 Std. zum Rückfluss (DC-Kontrolle). Nach beendeter Reaktion wird auf 15 ml 2N Schwefelsäure gegeben, kurz nachgerührt und dann werden die Phasen getrennt. Die org. Phase wird mit 5proz. Hydrogencarbonatlösung gewaschen, nach dem Trocknen, Einengen und Umkristallisieren aus Ethylacetat/n-Heptan erhält man das Produkt mit dem Molekulargewicht 337.4 (C₂₀H₁₉NO₄); MS (DCI+): 338 (M+H⁺). Durch Verwendung von optisch aktivem / enantiomer angereichertem 4-Phenyl-oxazolidin-2-on erhält man auf gleichem Weg optisch aktives / enantiomer angereichertes 2.

### b) 3-(5-Hydroxy-5-phenyl-pentanoyl)-4-phenyl-oxazolidin-2-on (3)

Zu einer Lösung von 1.5 ml Bordimethylsulfidkomplex in 25 ml Dichlormethan gibt man unter Argon bei einer Temperatur zwischen 0° und -5°C 5g 1-(2-Oxo-4-phenyl-oxazolidin-3-yl)-5-phenyl-pentan-1,5-dion in 20 ml Dichlormethan langsam über ca. 3 Std. Man rührt 2 std. bei gleicher Temperatur nach und kontrolliert durch Dünnschichtchromatographie. Nach beendeter Umsetzung gibt man unter 0°C 2 ml Methanol und 1.5 ml 35proz. Wasserstoffperoxidlösung sowie 1.1 ml 3N Schwefelsäure dazu und rührt 15 min bei Raumtemp. nach. Nach Phasentrennung wird die organische nacheinander gewaschen mit: 2N Schwefelsäure, 5 %proz. Natriumbisulfitlösung, 10proz. Kochsalzlösung und dann getrocknet und eingeengt. Nach Chromatographie (SiO₂, Ethylacetat/ n-Heptan = 1:1 erhält man das Produkt mit dem Molekulargewicht 339.4 (C₂₀H2₁NO₄); MS (DCI+): 322 (M+H⁺-H₂O); (ESI+): 403 (M+Na⁺+CH₃CN), 362 (M+Na⁺). Durch Zusatz von optisch aktivem 1-Methyl-3,3-diphenyl-tetrahydro-pyrrolo[1,2-c][1,3,2]oxazaborol (S oder R, 0.75 ml ) zur Reaktionsmischung bei 0° bis -5°C vor der Zugabe von 1-(2-Oxo-4-phenyl-oxazolidin-3-yl)-5-phenyl-pentan-1,5-dion erhält man auf gleichem Weg 3 in diastereomer angereicherter Form.

### c) 4-[1-(4-Fluor-phenylamino)-2-(2-oxo-4-phenyl-oxazolidin-3-carbonyl)-5-phenyl-5-trimethylsilanyloxy-pentyl]-benzonitril (4)

3.3 g 3-(5-Hydroxy-5-phenyl-pentanoyl)-4-phenyl-oxazolidin-2-on und 3.93 g 4-[(4-Fluor-phenylimino)-methyl]-benzonitril werden in 55 ml Dichlormethan gelöst auf -10°C gekühlt und langsam mit 8.5 ml Diisopropylethylamin versetzt. Innerhalb 30 min gibt man dann 5.3 ml Chlor-trimethyl-silan so dazu, dass die Temperatur unter -5°C bleibt. Nach einer Std. kühlt man auf -30°C und setzt 1.1 ml Titantetrachlorid unter -25°C zu und rührt dann über Nacht bei dieser Temperatur. Nach beendeter Umsetzung gibt man tropfenweise 4 ml Eisessig bei -25°C zu, rührt 15 min nach, gibt bei 0°C auf 50 ml 7proz. Weinsäure und rührt 1 Std. nach, gibt dann 25 ml 20 proz. Natriumbisulfitlösung dazu und rührt weitere 45 min nach. Nach Phasentrennung wird die organische Phase mit ca. 40 ml Wasser gewaschen, getrocknet und auf ca. 15 ml eingeengt. Dann setzt man 2.7 ml Bistrimethylsilylacetamid zu und erwärmt 30 min zum Rückfluss. Nach Abkühlung auf Raumtemp. wird eingeengt und nach Kristallisation des Rückstands aus Ethylacetat/n-Heptan erhält man das Produkt mit dem Molekulargewicht 635.8 (C₃₇H₃₈FN₃O₄Si); MS (ESI+): 636 (M+H⁺).

### d) 4-[1-(4-Fluor-phenyl)-3-(3-hydroxy-3-phenyl-propyl)-4-oxo-azetidin-2-yl]-benzonitril (5)

2.7 g 4-[1-(4-Fluor-phenylamino)-2-(2-oxo-4-phenyl-oxazolidin-3-carbonyl)-5-phenyl-5- trimethylsilanyloxy-pentyl]-benzonitril werden in 30 ml tert-Butyl-methyl-ether mit 1.6 ml Bistrimethylsilylacetamid und 0.2 g Tetrabutylammoniumfluorid-Trihydrat 3 Std zum Rückfluss erwärmt. Nach Stehen über Nacht gibt man 0.2 ml Eisessig dazu, rührt 15 min und engt weitgehend ein. Dazu gibt man 15 ml einer Mischung aus Isopropanol/2N Schwefelsäure= 10:1 und rührt 1 Std. bei Raumtemp. Danach behandelt man mit etwas festem Natriumhydrogencarbonat, engt erneut weitgehend ein, nimmt den Rückstand in Ethylacetat auf und wäscht mit Wasser. Der Rückstand der getrockneten organischen Phase wird durch Säulenfiltration gereinigt (SiO₂, Ethylacetat/n-Heptan = 1:1). Man erhält das Produkt mit dem Molekulargewicht 400.5 (C₂₅H₂₁FN₂O₂); MS (DCI+): 401 (M+H⁺), 383 (M+H⁺-H₂O).

### e) 4-(4-Aminomethyl-phenyl)-1-(4-fluor-phenyl)-3-(3-hydroxy-3-phenyl-propyl)-azetidin-2-on (6)

930 mg 4-[1-(4-Fluor-phenyl)-3-(3-hydroxy-3-phenyl-propyl)-4-oxo-azetidin-2-yl]-benzonitril werden in 100 ml Ethanol gelöst mit 4 ml konz. Ammoniak versetzt und über Raney-Ni 20 Std. bei Raumtemp. und 20 bar Wasserstoff hydriert. Man saugt vom Katalysator ab, engt i.Vak. ein und erhält nach Chromatographie (SiO₂, Dichlormethan/Methanol = 10:1) das Produkt mit dem Molekulargewicht 404.5 (C₂₅H₂₅FN₂O₂); MS (DCl+): 405 (M+H⁺), 387 (M+H⁺-H₂O).

### f) 5-[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-pentansäure (7)

2g 5-(3-Amino-phenyl)-3-butyl-7-dimethylamino-3-ethyl-1,1-dioxo-2,3,4,5-tetrahydro-1 H-benzo[b]thiepin-4-ol , 3.4 g Hexandisäure, 1.04 g Dicyclohexyl-carbodiimid und 640 mg Benzotriazol-1-ol werden in 80 ml Tetrahydrofuran über Nacht bei Raumtemp. gerührt. Man engt ein, nimmt den Rückstand mit Ethylacetat auf, filtriert vom überschüssigen Harnstoff ab und wäscht mit Wasser. Der Rückstand der getrockneten organischen Phase wird durch Säulenfiltration gereinigt (SiO₂, Dichlormethan/Methanol = 20:1). Man erhält das Produkt mit dem Molekulargewicht 558.7 (C₃₀H₄₂N₂O₆S); MS (ESI+): 559 (M+H⁺).

### g) Hexandisäure [3-(3-butyl-7-dimethylamino-3-ethyl-4-hydroxy-1,1-dioxo-2,3,4,5-tetrahydro-1H-benzo[b]thiepin-5-yl)-phenyl]-amid 4-[1-(4-fluor-phenyl)-3-(3-hydroxy-3-phenyl-propyl)-4-oxo-azetidin-2-yl]-benzylamid (8)

83 mg 4-(4-Aminomethyl-phenyl)-1-(4-fluor-phenyl)-3-(3-hydroxy-3-phenyl-propyl)-azetidin-2-on, 115 mg 5-[3-(3-Butyl-7-dimethylamino-3-ethyl-4-hydroxy-1,1-dioxo-2,3,4,5-tetrahydro-1H- benzo[b]thiepin-5-yl)-phenylcarbamoyl]-pentansäure, 45 mg Dicyclohexyl-carbodiimid und 35 mg Benzotriazol-1-ol werden in 5 ml Tetrahydrofuran über Nacht bei Raumtemp. gerührt. Man engt i.Vak. ein und erhält nach Chromatographie (SiO₂, Dichlormethan/Methanol = 20:1) das Produkt mit dem Schmelzpunkt 150°C und dem Molekulargewicht 945.2 (C₅₅H₆₅FN₄O₇S); MS (ESI+): 945 (M+H⁺).

### Beispiel III

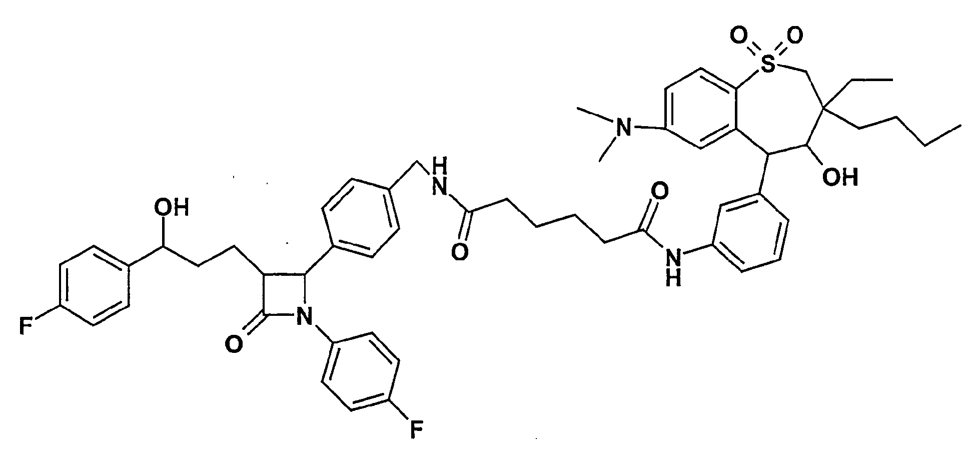

### Hexandisäure [3-(3-butyl-7-dimethylamino-3-ethyl-4-hydroxy-1,1-dioxo-2,3,4,5-tetrahydro-1H-benzo[b]thiepin-5-yl)-phenyl]-amid 4-{1-(4-fluor-phenyl)-3-[3-(4-fluor-phenyl)-3-hydroxy-propyl]-4-oxo-azetidin-2-yl}-benzylamid (12)

### a) 4-[5-(4-Fluor-phenyl)-1-(4-fluor-phenylamino)-2-(2-oxo-4-phenyl-oxazolidin-3-carbonyl)- 5-trimethylsilanyloxy-pentyl]-benzonitril (9)

Herstellung analog zu Beispiel II unter Verwendung von 3-[5-(4-Fluor-phenyl)-5-hydroxy-pentanoyl]-4-phenyl-oxazolidin-2-on
Produkt mit dem Molekulargewicht 653.8 (C₃₇H₃₇F₂N₃O₄Si); MS (ESI+): 654 (M+H⁺).

### b) 4-{1-(4-Fluor-phenyl)-3-[3-(4-fluor-phenyl)-3-hydroxy-propyl]-4-oxo-azetidin-2-yl}-benzonitril (10)

Herstellung analog Beispiel II, unter Verwendung von 4-[5-(4-Fluor-phenyl)-1-(4-fluor-phenylamino)-2-(2-oxo-4-phenyl-oxazolidin-3-carbonyl)-5-trimethylsilanyloxy-pentyl]-benzonitril ;Produkt mit dem Molekulargewicht 418.5 (C₂₅H₂₀F₂N₂O₂); MS (ESI+): 419 (M+H⁺).

### c) 4-(4-Aminomethyl-phenyl)-1-(4-fluor-phenyl)-3-[3-(4-fluor-phenyl)-3-hydroxypropyl]- azetidin-2-on (11)

Herstellung analog Beispiel II; unter Verwendung von 4-{1-(4-Fluor-phenyl)-3-[3-(4-fluor-phenyl)-3-hydroxy-propyl]-4-oxo-azetidin-2-yl}- benzonitril ;
Produkt mit dem Molekulargewicht 422.5 (C₂₅H₂₄F₂N₂O₂); MS (ESI+): 423 (M+H⁺).

### d) Hexandisäure [3-(3-butyl-7-dimethylamino-3-ethyl-4-hydroxy-1,1-dioxo-2,3,4,5-tetrahydro-1H-benzo[b]thiepin-5-yl)-phenyl]-amid 4-{1-(4-fluor-phenyl)-3-[3-(4-fluorphenyl)-3-hydroxy-propyl]-4-oxo-azetidin-2-yl}-benzylamid (12)

Herstellung analog Beispiel II; Produkt mit dem Molekulargewicht 963.2 (C₅₅H₆₄F₂N₄O₇S); MS (ESI+): 963 (M+H⁺).

### Beispiel V

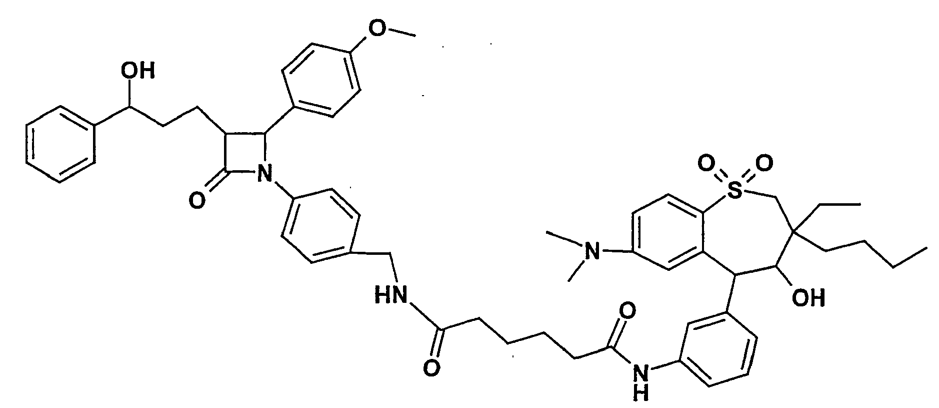

### Hexandisäure [3-(3-butyl-7-dimethylamino-3-ethyl-4-hydroxy-1,1-dioxo-2,3,4,5-tetrahydro-1H-benzo[b]thiepin-5-yl)-phenyl]-amid 4-[3-(3-hydroxy-3-phenyl- propyl)-2-(4-methoxy-phenyl)-4-oxo-azetidin-1-yl]-benzylamid (15)

Herstellung analog Beispiel III, ausgehend von 1-(4-Aminomethyl-phenyl)-3-(3-hydroxy-3-phenyl-propyl)-4-(4-methoxy-phenyl)-azetidin-2- on;
Produkt mit dem Molekulargewicht 957.2 (C₅₆H₆₈N₄O₈S); MS (ESI+): 957 (M⁺H⁺).

### Beispiel VI

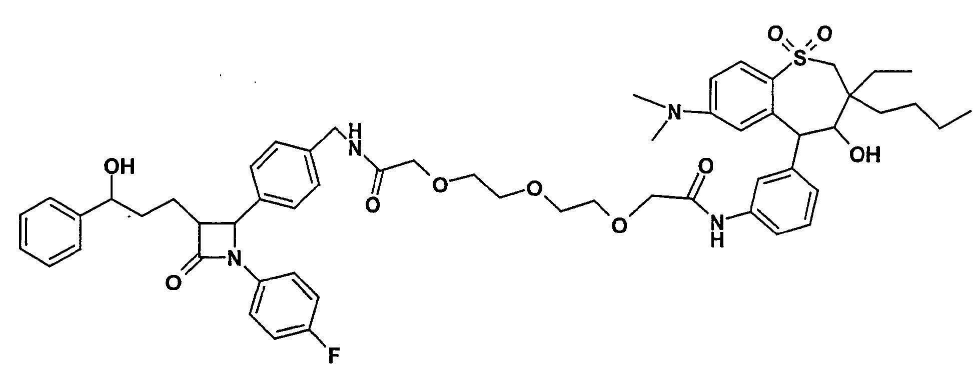

### [2-(2-{[3-(3-Butyl-7-dimethylamino-3-ethyl-4-hydroxy-1,1-dioxo-2,3,4,5-tetrahyd ro-1H- benzo[b]thiepin-5-yl)-phenylcarbamoyl]-methoxy}-ethoxy)-ethoxy]-[N-{4-[1-(4-fluor-phenyl)-3-(3-hydroxy-3-phenyl-propyl)-4-oxo-azetidin-2-yl]-benzyl}]-acetamid (16)

Herstellung analog Beispiel II, ausgehend von 83 mg 4-(4-Aminomethyl-phenyl)-1-(4-fluor-phenyl)-3-(3-hydroxy-3-phenyl-propyl)-azetidin-2- on und 130 mg [2-(2-{[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-methoxy}-ethoxy)-ethoxy]-essigsäure; Chromatographie: SiO₂, Dichlormethan/Methanol = 20:1 ;
Produkt mit dem Schmelzpunkt 120°C und dem Molekulargewicht 1021.3 (C₅₇H₆₇FN₄O₁₀S); MS (ESI+): 1021 (M+H⁺).

### Beispiel VII

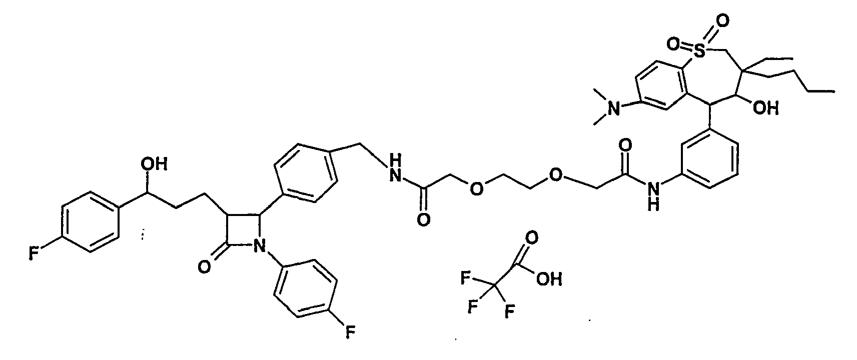

### (3-Butyl-3-ethyl 5-[3-(2-{2-[(4-{1-(4-fluor-phenyl)-3-[3-(4-fluor-phenyl)-3-hydroxypropyl]-4-oxo- azetidin-2-yl}-benzylcarbamoyl)-methoxy]-ethoxy}-acetylamino)-phenyl]-4-hydroxy-1,1-dioxo- 2,3,4,5-tetrahydro-1H-benzo[b]thiepin-7-yl)-dimethyl-ammonium; trifluor-acetat (18)

### a) (2-{[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-methoxy}-ethoxy)-essigsäure (17)

Zu einer Lösung von 965 mg 10 g Dioxaoctandisäure, 188 mg Hydroxybenzotriazol und 287 mg Dicyclohexylcarbodiimid in 10 ml Tetrahydrofuran (THF) werden über 2 h 500 mg 5-(3-Amino-phenyl)-3-butyl-7-dimethylamino-3-ethyl-1,1-dioxo-2,3,4,5-tetrahydro-1H- 1-benzo[b]thiepin-4-ol in 8 ml THF zugetropft. Es wird bei Raumtemperatur über 12 h gerührt. Die Reaktionslösung wird eingeengt, mit 2 N Salzsäure aufgenommen und mit Ethylacetat extrahiert. Die organische Phase wird über Magnesiumsulfat getrocknet, eingeengt und über HPLC (Merk-Hibar-Lichrospher 100-RP-18, Wasser (0.1 % Trifluoressigsäure)/Acetonitril (0.1% Trifluoressigsäure) = 80/20 -> 10/90) gereinigt. Man erhält 17.
C₃₀H₄₁N₂O₈S₁ (590.74) MS (ESI) 592 (M + H)

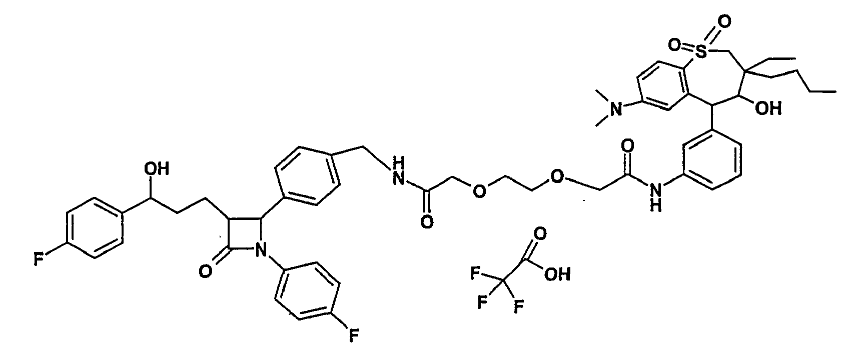

### b) (3-Butyl-3-ethyl-5-[3-(2-{2-[(4-{1-(4-fluor-phenyl)-3-[3-(4-fluor-phenyl)-3-hydroxypropyl]-4-oxo- azetidin-2-yl}-benzylcarbamoyl)-methoxy]-ethoxy}-acetylamino)-phenyl]-4-hydroxy-1,1-dioxo- 2,3,4,5-tetrahydro-1H-benzo[b]thiepin-7-yl)-dimethyl-ammonium; trifluor-acetat (18)

Eine Lösung aus 100 mg 4-(4-Aminomethyl-phenyl)-1-(4-fluor-phenyl)-3-[3-(4-fluorophenyl)-3-hydroxy-propyl]- azetidin-2-on, 209 mg (2-{[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-methoxy}-ethoxy)-essigsäure , 93 µl Diisopropylcarbodiimid, 65 mg Hydroxybenzotriazol in 2 ml Methylenchlorid wird 12 h bei Raumtemperatur gerührt. Es wird Wasser zugegeben und mit Methylenchlorid extrahiert. Die organische Phase wird über Magnesiumsulfat getrocknet, eingeengt und über HPLC (Knauer Eurospher-100-10-C18, Wasser (0.1 % Trifluoressigsäure)/Acetonitril (0.1% Trifluoressigsäure) = 80/20 -> 10/90) getrennt. Man erhält 18.
C₅₇H₆₃F₅N₄O₁₁S₁ (1109.23) MS (ESI) 977 (M + H - H₂O)

Analog zu Beispiel VII werden folgende Beispiele (VIII-XXIV) hergestellt:

### Beispiel VIII

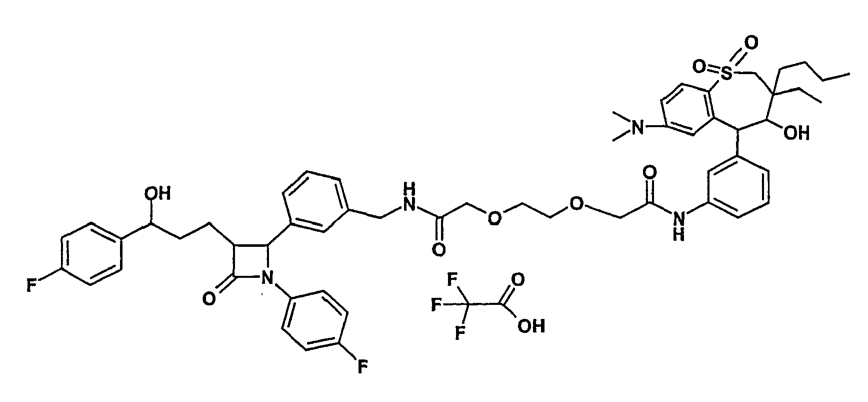

### (3-Butyl-3-ethyl 5-[3-(2-{2-[(3-{1-(4-fluor-phenyl)-3-[3-(4-fluor-phenyl)-3-hydroxy-propyl]-4-oxo- azetidin-2-yl}-benzylcarbamoyl)-methoxy]-ethoxy}-acetylamino)-phenyl]- 4-hydroxy-1,1-dioxo-2,3,4,5-tetrahydro-1H-benzo[b]thiepin-7-yl)-dimethyl-ammonium; trifluor-acetat (19)

C₅₇H₆₅F₅N₄O₁₁S₁ (1109.23) MS (ESI) 977 (M + H - H₂O)

### Beispiel IX

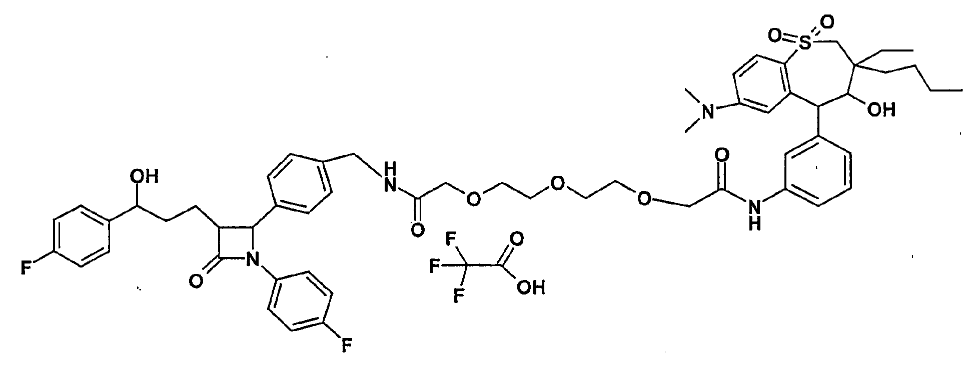

### 3-Butyl-3-ethyl-5-{3-[2-(2-{2-[(4-{1-(4-fluor-phenyl)-3-[3-(4-fluor-phenyl)-3-hydroxypropyl]-4-oxo-azetidin-2- yl}-benzylcarbamoyl)-methoxy]-ethoxy}-ethoxy)-acetylamino]-phenyl}-4-hydroxy-1,1-dioxo- 2,3,4,5-tetrahydro-1H-benzo[b]thiepin-7-yl)-dimethyl-ammonium; trifluor-acetat (21)

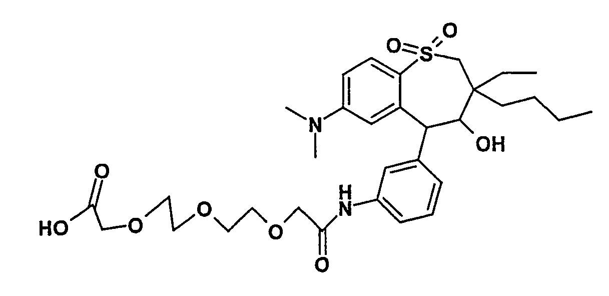

### a) [2-(2-{[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-methoxy}-ethoxy)-ethoxy]-essigsäure (20)

C₃₂H₄₆N₂O₃S₁ (634.3) MS (ESI) 635 (M + H)

b) (3-Butyl-3-ethyl-5-{3-[2-(2-{2-[(4-{1-(4-fluor-phenyl)-3-[3-(4-fluor-phenyl)-3- 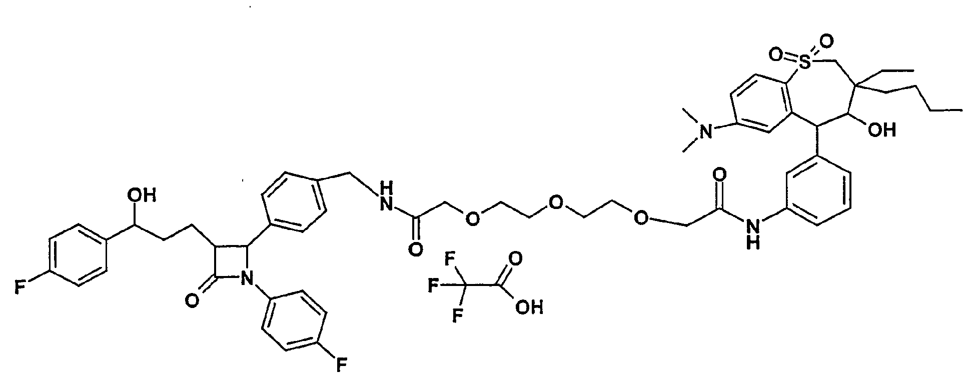 hydroxy-propyl]-4-oxo-azetidin-2- yl}-benzylcarbamoyl)-methoxy]-ethoxy}-ethoxy)-acetylamino]-phenyl}-4-hydroxy-1,1-dioxo- 2,3,4,5-tetrahydro-1H-benzo[b]thiepin-7-yl)-dimethyl-ammonium; trifluor-acetat (21)
C₅₉H₆₉F₅N₄O₁₂S₁ (1153,28) MS (ESI) 1039 (M + H)

### Beispiel X

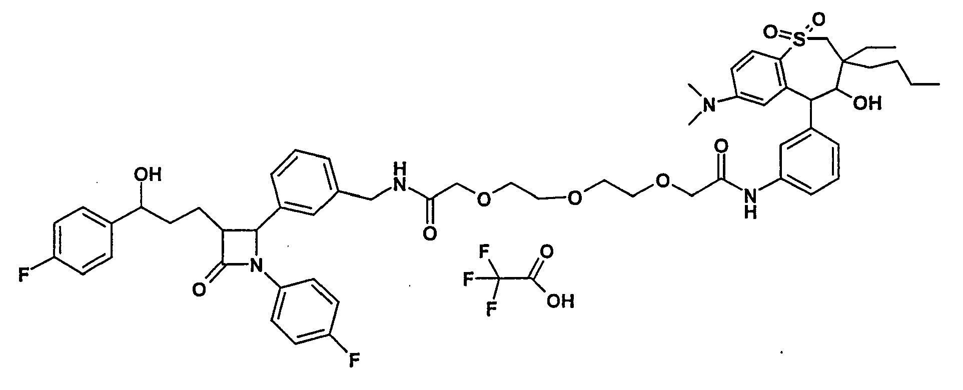

### (3-Butyl-3-ethyl-5-{3-[2-(2-{2-[(3-{1-(4-fluor-phenyl)-3-[3-(4-fluor-phenyl)-3-hydroxy-propyl]-4-oxo-azetidin-2-yl}-benzylcarbamoyl)-methoxy]-ethoxy}-ethoxy)-acetylamino]-phenyl}-4-hydroxy-1,1-dioxo-2,3,4,5-tetrahydro-1H-benzo[b]thiepin-7-yl)-dimethyl-ammonium; trifluor-acetat (22)

C₅₉H₆₉F₅N₄O₁₂S₁ (1153.28) MS (ESI) 1040 (M + H)

### Beispiel XI

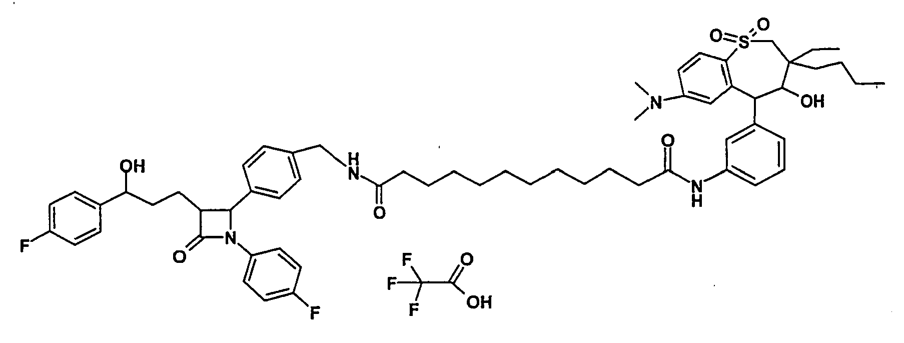

### (3-Butyl-3-ethyl-5-{3-[11-(4-{1-(4-fluor-phenyl)-3-[3-(4-fluor-phenyl)-3-hydroxy-propyl]-4- oxo-azetidin-2-yl}-benzylcarbamoyl)-undecanoylamino]-phenyl}-4-hydroxy-1,1-dioxo-2,3,4,5-tetrahydro-1H-benzo[b]thiepin-7-yl)-dimethyl-ammonium; trifluor-acetat (24)

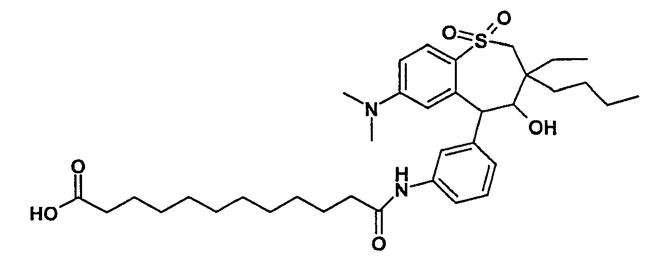

### a) 11-[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-undecansäure (23)

C₃₆H₅₄N₂O₆S₁ (642.91) MS (ESI) 643 (M + H)

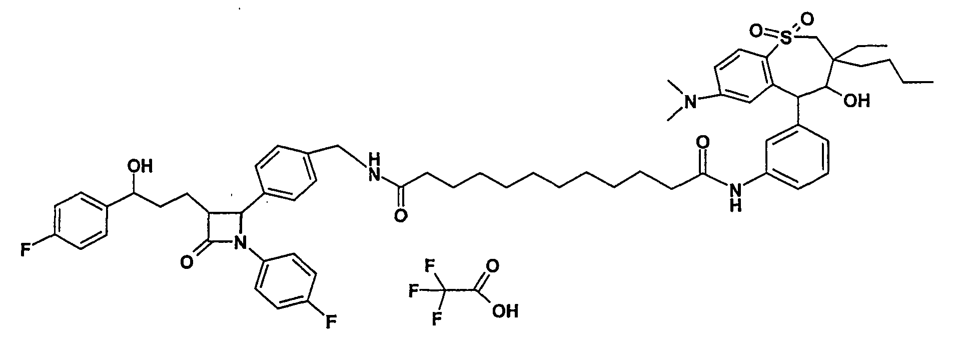

### b) (3-Butyl-3-ethyl-5-{3-[11-(4-{1-(4-fluor-phenyl)-3-[3-(4-fluor-phenyl)-3-hydroxy-propyl]-4- oxo-azetidin-2-yl}-benzylcarbamoyl)-undecanoylamino]-phenyl}-4-hydroxy-1,1-dioxo-2,3,4,5- tetrahydro-1H-benzo[b]thiepin-7-yl)-dimethyl-ammonium; trifluor-acetat (24)

C₆₃H₇₇F₅N₄O₉S₁ (1161,39) MS (ESI) 1047 (M + H)

### Beispiel XII

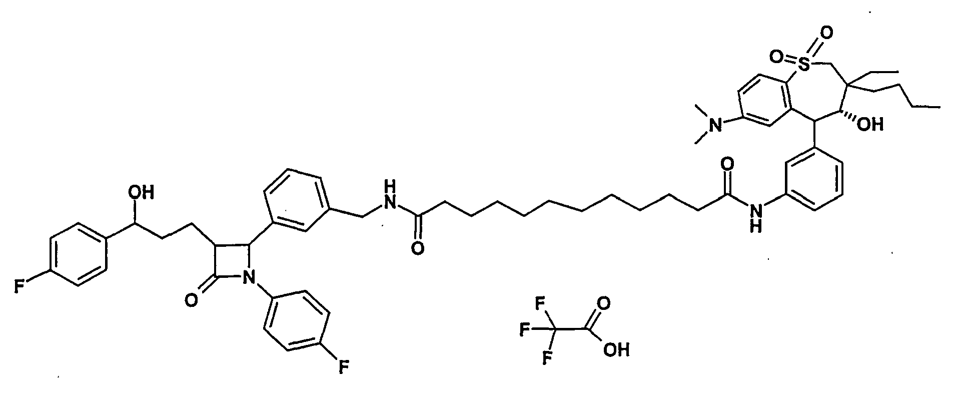

### (3-Butyl-3-ethyl-5-{3-[11-(3-{1-(4-fluor-phenyl)-3-[3-(4-fluor-phenyl)-3-hydroxy-propyl]-4- oxo-azetidin-2-yl}-benzylcarbamoyl)-undecanoylamino]-phenyl}-4-hydroxy-1,1-dioxo-2,3,4,5- tetrahydro-1H-benzo[b]thiepin-7-yl)-dimethyl-ammonium; trifluor-acetat (25)

C₆₃H₇₇F₅N₄O₉S₁ (1161.39) MS (ESI) 1047 (M + H)

### Beispiel XXI

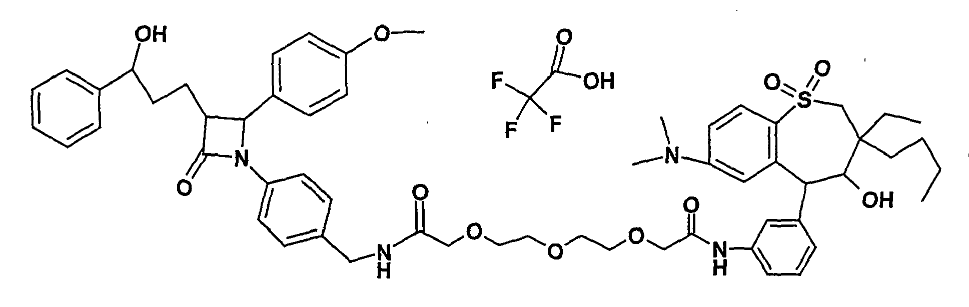

### (3-Butyl-3-ethyl-5-{3-[2-(2-{2-[(4-[3-(3-hydroxy-3-phenyl-propyl)-2-(4-methoxy-phenyl)-4-oxo-azetidin-1-yl]- benzylcarbamoyl)-methoxy]-ethoxy}-ethoxy)-acetylamino]-phenyl}-4-hydroxy-1,1-dioxo-2,3,4,5-tetrahydro-1H-benzo[b]thiepin- 7-yl)-dimethyl-ammonium; trifluor-acetat (38)

C₆₀H₇₃F₃N₄O₁₃S₁ (1147.33) MS (ESI) 1033 (M + H)

### Beispiel XXII

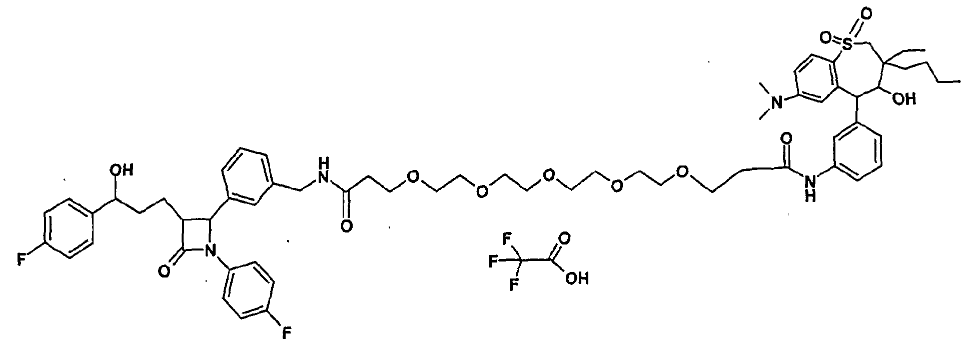

### {3-Butyl-3-ethyl-5-[3-(3-{2-[2-(2-{2-[2-(3-{1-(4-fluor-phenyl)-3-[3-(4-fluor-phenyl)-3-hydroxy-propyl]-4- oxo-azetidin-2-yl}-benzylcarbamoyl)-ethoxy]-ethoxy}- ethoxy)-ethoxy]-ethoxy}-propionylamino)-phenyl]-4-hydroxy-1,1-dioxo-2,3,4,5-tetrahydro- 1H-benzo[b]thiepin-7-yl}-dimethyl-ammonium; trifluor-acetat (42)

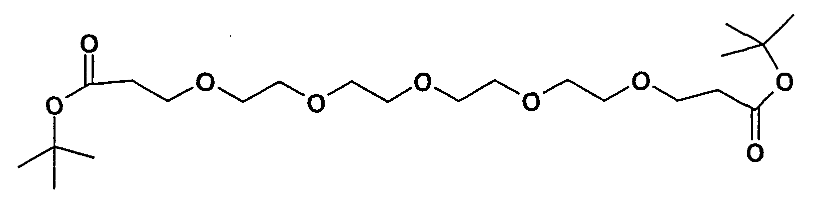

### a) 3-[2-(2-{2-[2-(2-tert-Butoxycarbonyl-ethoxy)-ethoxy]-ethoxy}-ethoxy)-ethoxy]-propionsäure-tert-butyl ester (39)

Zu einer Lösung von 91 g Tetraethylenglycol in 250 ml Tetrahydrofuran werden 0.4 g Natrium gegeben und bei Raumtemperatur gerührt. Wenn sich das Natrium aufgelöst hat wird 145 ml tert.-Butylacrylat zugegeben. Man rührt 12 h. Die Reaktionslösung wird mit Ammoniumchlorid neutralisiert, eingeengt , in wässriger Natriumchloridlösung aufgenommen und mit Ethylacetat extrahiert. Die organische Phase wird eingeengt. Der Rückstand ist 39.

C₂₂H₄₂O₉ (450.57) MS (ESI) 339 (M + 3*H - 2* tert-Bu)

### b) 3-[2-(2-{2-[2-(2-Carboxy-ethoxy)-ethoxy]-ethoxy}-ethoxy)-ethoxy]-propionsäure (40)

Eine Lösung von 3-[2-(2-{2-[2-(2-tert-Butoxycarbonyl-ethoxy)-ethoxy]-ethoxy}-ethoxy)-ethoxy]-propionsäure-tert-butyl ester 24 in 50 ml Methylenchlorid mit 50 ml Trifluoressigsäure wird 2 h gerührt und eingeengt. Der Rückstand wird in 1N-Salzsäure aufgenommen und mit Methylenchlorid extrahiert. Die organische Phase wird eingeengt und enthält 40.
C₁₄H₂₆O₉ (338.36) MS (ESI) 339 (M + H)

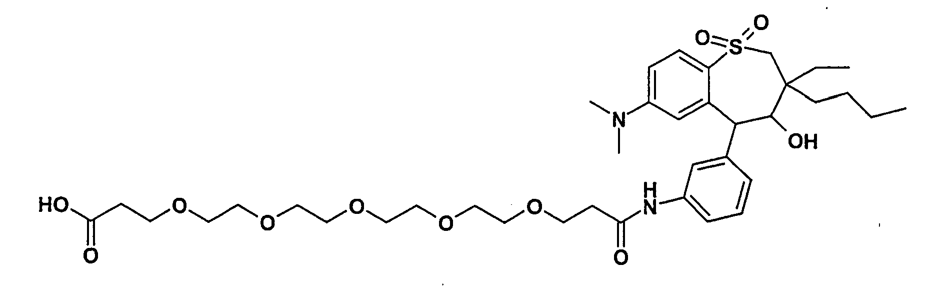

### c) 3-(2-{2-[2-(2-{2-[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-ethoxy}-ethoxy)-ethoxy]-ethoxy}-ethoxy)-propionsäure (41)

Synthese erfolgt analog zu 17.
C₃₉H₆₀N₂O₁₁S₁ (750.97) MS (ESI) 751 (M + H)

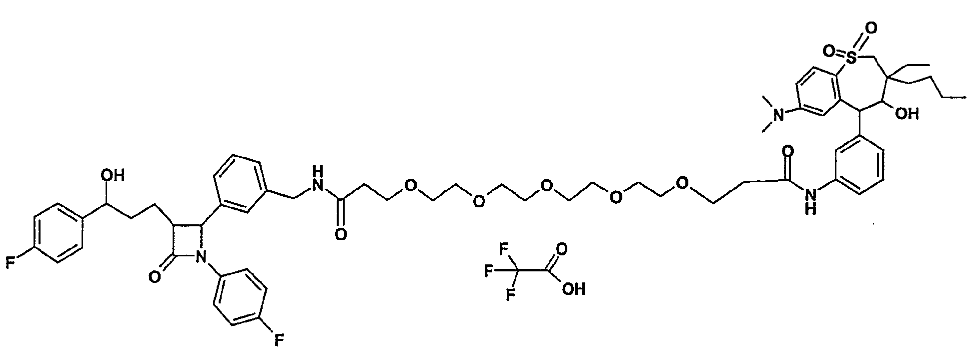

### d) {3-Butyl-3-ethyl-5-[3-(3-{2-[2-(2-{2-[2-(3-{1-(4-fluor-phenyl)-3-[3-(4-fluor-phenyl)-3-hydroxy-propyl]-4- oxo-azetidin-2-yl}-benzylcarbamoyl)-ethoxy]-ethoxy}- ethoxy)-ethoxy]-ethoxy}-propionylamino)-phenyl]-4-hydroxy-1,1-dioxo-2,3,4,5-tetrahydro- 1H-benzo[b]thiepin-7-yl}-dimethyl-ammonium; trifluor-acetat (42)

C₆₅H₈₁F₅N₄O₁₄S₁ (1269.44) MS (ESI) 1155 (M + H)

### Beispiel XXIII

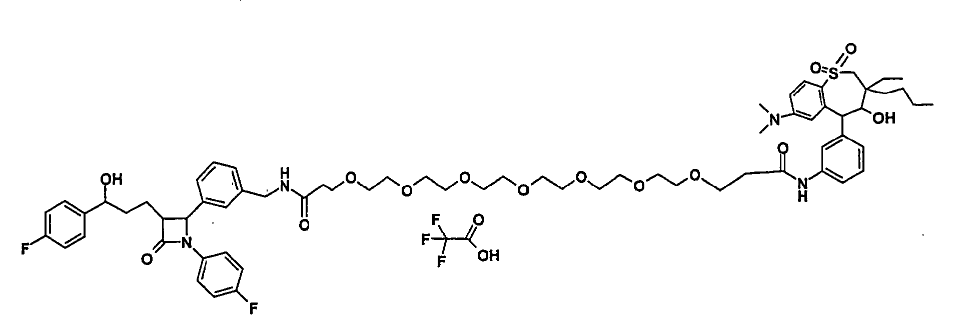

### [3-Butyl-3-ethyl-5-(3-{3-[2-(2-{2-[2-(2-{2-[2-{3-[1-(4-fluor-phenyl)-3-[3-(4-fluor-phenyl)-3-hydroxy-propyl]-4-oxo-azetidin-2- yl]-benzylcarbamoyl}-ethoxy]-ethoxy}- ethoxy)-ethoxy]-ethoxy}-ethoxy)-ethoxy]-propionylamino}-phenyl)-4-hydroxy-1,1-dioxo- 2,3,4,5-tetrahydro-1H-benzo[b]thiepin-7-yl]-dimethyl-ammonium; trifluoracetat (46)

### a) 3-(2-{2-[2-(2-{2-[2-(2-tert-Butoxycarbonyl-ethoxy)-ethoxy]-ethoxy}-ethoxy)-ethoxy]-ethoxy}-ethoxy)-propionsäure- tert-butyl ester (43)

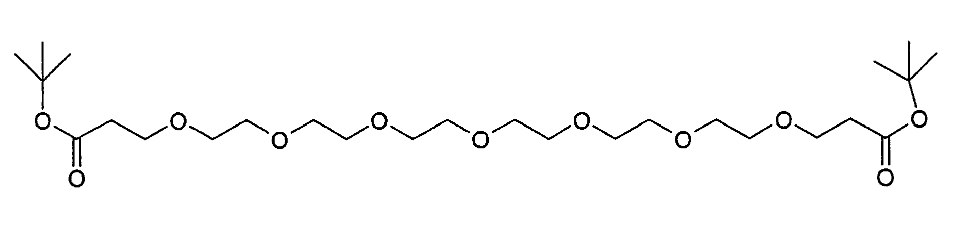

Synthese erfolgt analog zu 39.
C₂₆H₅₀O₁₁ (538.68) MS (ESI) 427 (M + 3*H - 2* tert-Bu)

### b) 3-(2-{2-[2-(2-{2-[2-(2-Carboxy-ethoxy)-ethoxy]-ethoxy}-ethoxy)-ethoxy]-ethoxy}-ethoxy)- propionsäure (44)

Synthese erfolgt analog zu 40.
C₁₈H₃₄O₁₁ (426.47) MS (ESI) 427 (M + H)

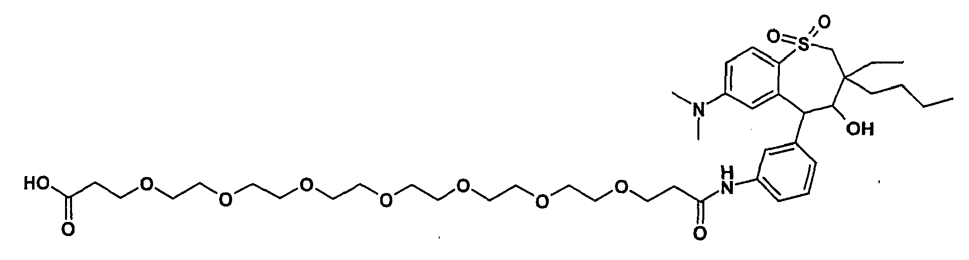

### c) 3-{2-[2-(2-{2-[2-(2-{2-[3-(3-Butyl-7-dimethylamino-3-ethyl-4-hydroxy-1,1-dioxo-2,3,4,5- tetrahydro-1H-benzo[b]thiepin-5-yl)-phenylcarbamoyl]-ethoxy}-ethoxy)-ethoxy]- ethoxy}-ethoxy)-ethoxy]-ethoxy}-propionsäure (45)

Synthese erfolgt analog zu 17.
C₄₃H₆₆N₂O₃S₁ (839.09) MS (ESI) 840 (M + H)

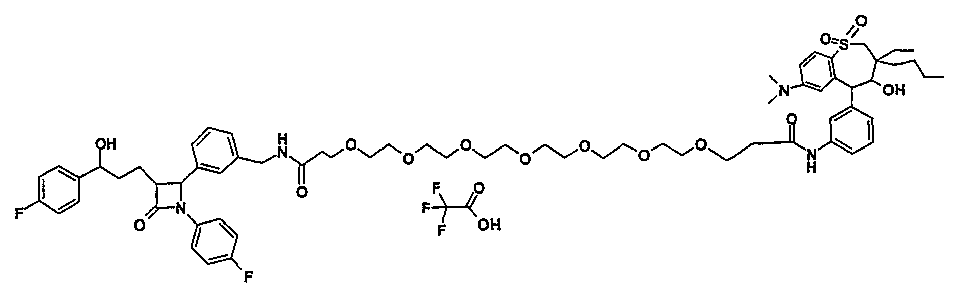

### d) [3Butyl-3-ethyl-5-(3-{3-[2-(2-{2-[2-(2-{2-[2-{3-[1-(4-fluoro-phenyl)-3-[3-(4-fluoro-phenyl)-3-hydroxy-propyl]-4-oxo-azetidin-2-yl]-benzylcarbamoyl}-ethoxy]-ethoxy}-ethoxy)-ethoxy]-ethoxy}-ethoxy)-ethoxy]-propionylamino}-phenyl)-4-hydroxy-1,1-dioxo- 2,3,4,5-tetrahydro-1H-benzo[b]thiepin-7-yl]-dimethyl-ammonium; trifluor-acetat (46)

C₆₉H₈₉F₅N₄O₁₆S₁ (1357.55) MS (ESI) 1243 (M + H)

### Beispiel XXIV

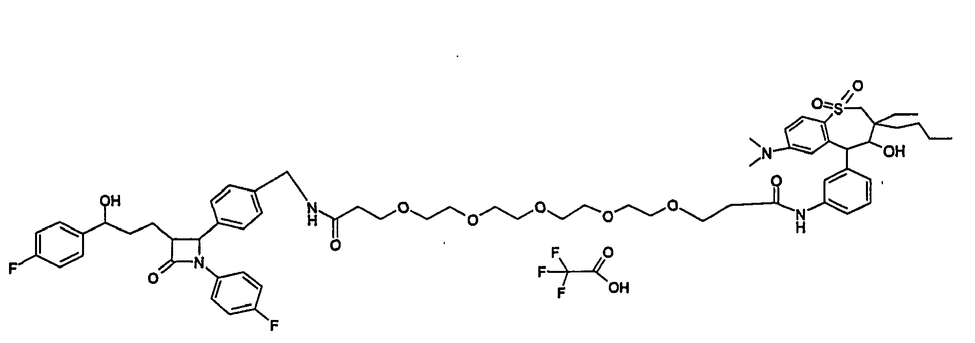

### [3-Butyl-3-ethyl-5-(3-{3-[2-(2-{2-[2-({2-{2-[2-(4-{1-(4-Fluor-phenyl)-3-[3-(4-fluor-phenyl)-3-hydroxy-propyl]-4-oxo-azetidin-2-yl}-benzylcarbamoyl}-ethoxy)-ethoxy]-ethoxy}-ethoxy)-ethoxy]-propionylamino}-phenyl)-4-hydroxy-1,1-dioxo-2,3,4,5-tetrahydro-1H-benzo[b]thiepin-7-yl]-dimethyl-ammonium; trifluor-acetat (47)

C₆₅H₈₁F₅N₄O₁₄S₁ (1269.44) MS (ESI) 1243 (M + H)

### Beispiel XXV

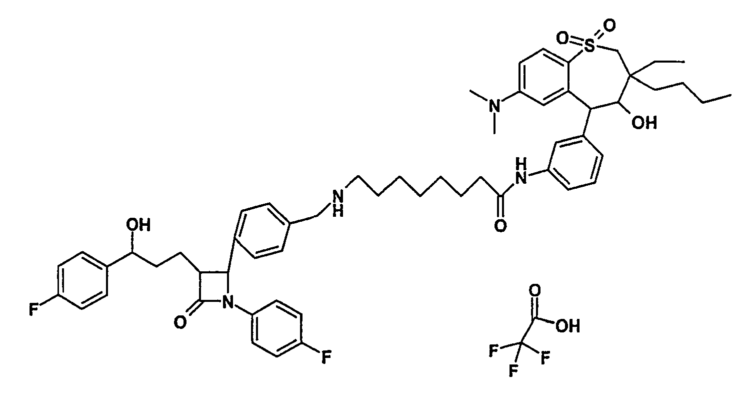

### (3-Butyl-3-ethyl-5-{3-[8-(4-{1-(4-fluor-phenyl)-3-[3-(4-fluor-phenyl)-3-hydroxy-propyl]-4-oxo-azetidin-2-yl}-benzylamino)-octanoylamino]-phenyl}-4-hydroxy-1,1-dioxo-2,3,4,5- tetrahydro-1H-benzo[b]thiepin-7-yl)-dimethyl-ammonium; trifluor-acetat (50)

### a) 7-[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-heptansäure (48)

Synthese erfolgt analog 17.
C₃₃H₄₈N₂O₆S₁ (600.82) MS (ESI) 601 (M + H)

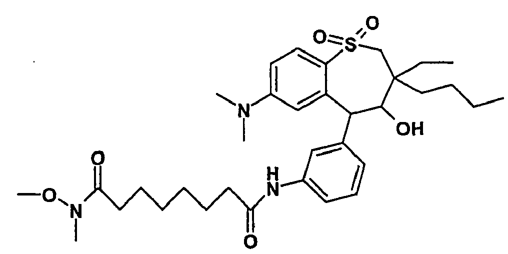

### b) Octandisäure- [3-(3-butyl-7-dimethylamino-3-ethyl-4-hydroxy-1,1-dioxo-2,3,4,5-tetrahydro-1H-benzo[b]thiepin-5-yl)-phenyl]-amid methoxy-methyl- amid (49)

Zu einer Lösung von 550 mg 7-[3-(3-Butyl-7-dimethylamino-3-ethyl-4-hydroxy-1,1-dioxo-2,3,4,5-tetrahydro-1-benzo[b]thiepin-5-yl)-phenylcarbamoyl]-heptansäure, 311 µl Diisopropylcarbodiimid, 272 mg Hydroxybenzotriazol in 10 ml Methylenchlorid werden bei Raumtemperatur eine Lösung aus 223 mg O,N-Dimethyl-hydroxylamin hydrochlorid und 391 µl Diisopropylethylamin in 5 ml Acetonitril gegebenen und 12 h gerührt. Die Reaktionslösung wird eingeengt und über HPLC (Merck-Hibar-Lichrospher 100-RP-18, Wasser (0.1 % Trifluoressigsäure)/Acetonitril (0.1% Trifluoressigsäure) = 80/20 -> 10/90) gereinigt.
C₃₅H₅₃N₃O₆S₁ (643.89) MS (ESI) 644 (M + H)

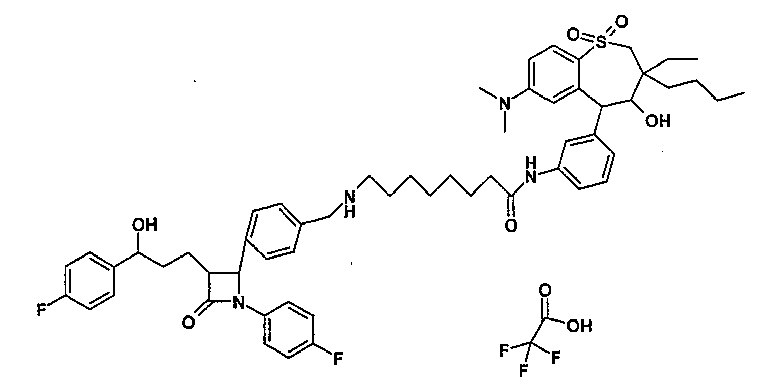

### c) (3-Butyl-3-ethyl-5-{3-[8-(4-{1-(4-fluor-phenyl)-3-[3-(4-fluor-phenyl)-3-hydroxy-propyl]- 4-oxo-azetidin-2-yl}-benzylamino)-octanoylamino]-phenyl}-4-hydroxy-1,1-dioxo-2,3,4,5- tetrahydro-1H-benzo[b]thiepin-7-yl)-dimethyl-ammonium; trifluoro-acetat (50)

Zu einer Lösung aus 160 mg Octandisäure-[3-(3-butyl-7-dimethylamino-3-ethyl-4-hydroxy-1,1-dioxo -2,3,4,5- tetrahydro-1H-benzo[b]thiepin-5-yl)-phenyl]-amid methoxy-methyl-amid 34 in 1 ml Tetrahydrofuran werden bei -78°C mit 0.22 ml einer 1M Lösung von Diisobutylaluminiumhydrid in Hexan versetzt. und 30 min gerührt. Die Reaktionslösung wird mit Wasser versetzt und mit Methylenchlorid extrahiert. Es wird eingeengt und in 3 ml einer Mischung aus Tetrahydrofuran und Methanol (1/1, 1 % Essigsäure) aufgenommen. Dazu gibt man 131 mg 4-(3-Aminomethyl-phenyl)-1-(4-fluor-phenyl)-3-[3-(4-fluor-phenyl)-3-hydroxy-propyl]- azetidin-2-on und 58 mg Natriumcyanoborhydrid. Nach 12 h wird die Reaktion mit Wasser versetzt, mit Methylenchlorid extrahiert und die organische Phase eingeengt. Der Rückstand wird über HPLC (Knauer Eurospher-100-10-C18, Wasser (0.1 % Trifluoressigsäure)/Acetonitril (0.1% Trifluoressigsäure) = 80/20 -> 10/90) gereinigt.
C₅₈H₇₂F₂N₄O₆S₁ (991.30) MS (ESI) 991 (M + H)

### Beispiel XXVI

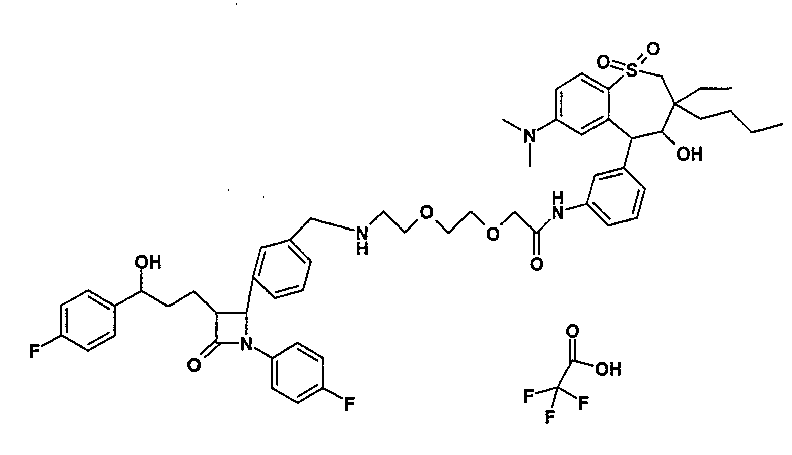

### {3-Butyl-3-ethyl-5-[3-(2-{2-[2-(3-{1-(4-fluor-phenyl)-3-[3-(4-fluor-phenyl)-3-hydroxy-propyl]-4-oxo- azetidin-2-yl}-benzylamino)-ethoxy]-ethoxy}-acetylamino)-phenyl]-4-hydroxy-1,1-dioxo- 2,3,4,5-tetrahydro-1H-benzo[b]thiepin-7-yl}-dimethyl-ammonium; trifluor- acetat (52)

### a) 2-(2-{[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-methoxy}-ethoxy)-N-methoxy-N-methyl-acetamid (51)

Synthese analog zu 49 ausgehend von 17.
C₃₂H₄₇N₃O₈S₁ (633.81) MS (ESI) 634 (M + H)

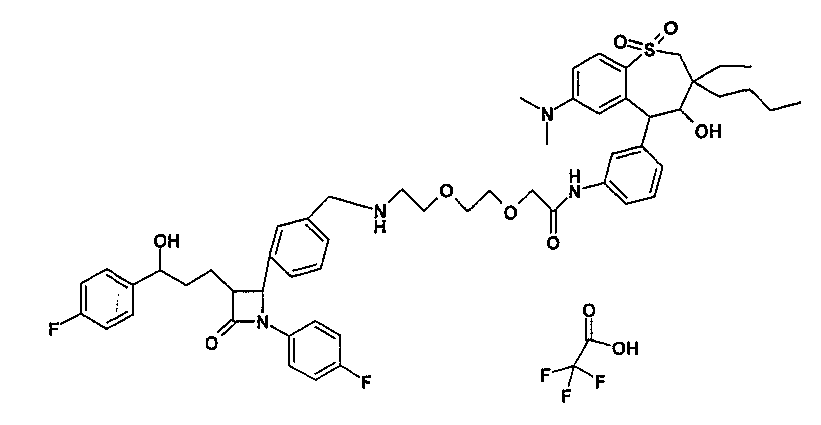

### b) {3-Butyl-3-ethyl-5-[3-(2-{2-[2-(3-{1-(4-fluor-phenyl)-3-[3-(4-fluor-phenyl)-3-hydroxy-propyl]-4-oxo- azetidin-2-yl}-benzylamino)-ethoxy]-ethoxy}-acetylamino)-phenyl]-4-hydroxy-1,1-dioxo- 2,3,4,5-tetrahydro-1H-benzo[b]thiepin-7-yl}-dimethyl-ammonium; trifluor- acetat (52)

Synthese analog 50.
C₅₇H₆₇F₅N₄O₁₀S₁ (1095.25) MS (ESI) 982 (M + H)

### Beispiel XXVII

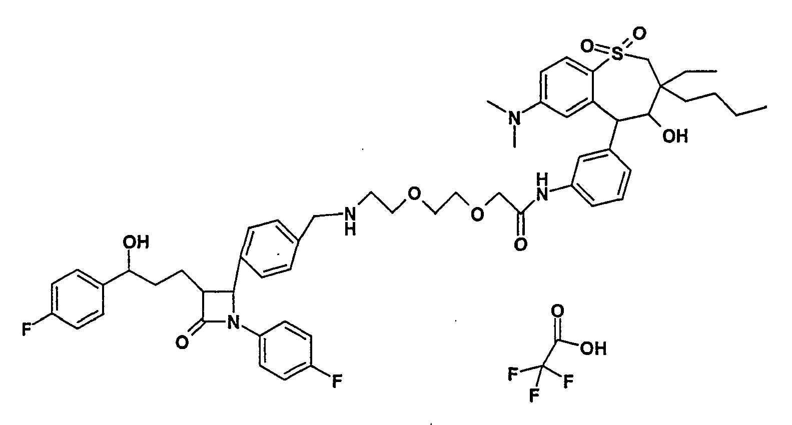

### {3-Butyl-3-ethyl-5-[3-(2-{2-[2-(4-{1-(4-fluor-phenyl)-3-[3-(4-fluor-phenyl)-3-hydroxy-propyl]-4-oxo- azetidin-2-yl}-benzylamino)-ethoxy]-ethoxy}-acetylamino)-phenyl]-4-hydroxy-1,1-dioxo- 2,3,4,5-tetrahydro-1H-benzo[b]thiepin-7-yl}-dimethyl-ammonium; trifluor- acetat (53)

Synthese analog 50.
C₅₇H₆₇F₅N₄O₁₀S₁ (1095.25) MS (ESI) 982 (M + H)

### Beispiel XXVIII

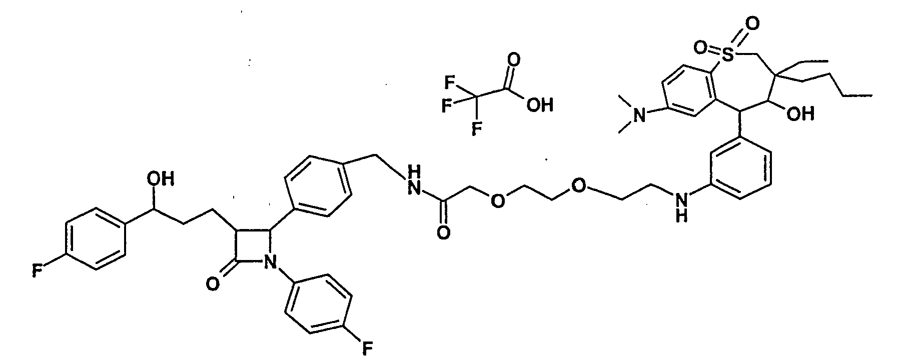

### {3-Butyl-3-ethyl-5-[3-(2-{2-[(4-{1-(4-fluor-phenyl)-3-[3-(4-fluor-phenyl)-3-hydroxy-propyl]-4-oxo- azetidin-2-yl}-benzylcarbamoyl)-methoxy]-ethoxy}-ethylamino)-phenyl]-4-hydroxy-1,1-dioxo- 2,3,4,5-tetrahydro-1H-benzo[b]thiepin-7-yl}-dimethyl-ammonium; trifluor- acetat (58)

### a) {2-[(Methoxy-methyl-carbamoyl)-methoxy]-ethoxy}-essigsäure (54)

Zu einer Lösung von 10 g Dioxaoctandisäure, 13 ml Diisopropylcarbodiimid, 11.4 g Hydroxybenzotriazol in 70 ml Methylenchlorid wird eine Lösung aus 5.5 g O,N-Dimethyl-hydroxylamine hydrochlorid und 9.6 ml Diisopropylethylamin in 50 ml Acetonitril und 40 ml DMF gegebenen und 12 h gerührt. Die Reaktionslösung wird eingeengt, und über eine Chromatographie an Kieselgel (Ethylacetat/Heptan/Methanol/Essigsäure = 8/10/1/1 -> 0/0/10/1) gereinigt.
C₈H₁₉N₁O₄ (221.21) MS (ESI) 222 (M + H)

### b) {2-[(Methoxy-methyl-carbamoyl)-methoxy]-ethoxy}-essigsäure tert-butyl ester (55)

Zu einer Lösung von 2 g {2-[(Methoxy-methyl-carbamoyl)-methoxy]-ethoxy}-essigsäure 39 in 20 ml Methylenchlorid wird 1.3 ml Thionylchlorid gegeben und 1 h bei 60°C gerührt. Nach Zugabe von 1.3 ml tert.-Butanol wird weitere 2 h bei Raumtemperatur gerührt. Man versetzt mit Wasser, extrahiert mit Methylenchlorid, engt ein und erhält 55.
C₁₂H₂₃N₁O₆ (277.32) MS (ESI) 222 (M + 2*H - tert.-butyl)

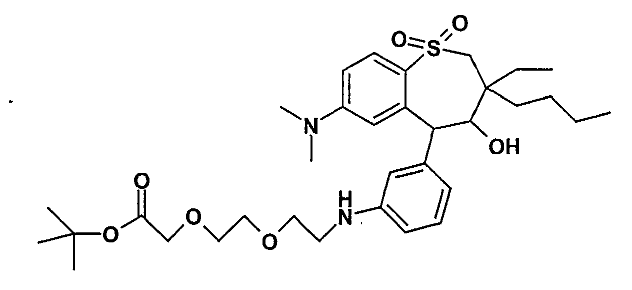

### c) (2-{2-[3-(3-Butyl-7-dimethylamino-3-ethyl-4-hydroxy-1,1-dioxo-2,3,4,5-tetrahydro-1H-benzo[b]thiepin-5-yl)-phenylamino]-ethoxy}-ethoxy)-essigsäure tert-butyl ester (56)

Synthese analog 50 ausgehend von 55 und 5-(3-Amino-phenyl)-3-butyl-7-dimethylamino-3-ethyl-1,1-dioxo-2,3,4,5-tetrahydro-1H-benzo[b]thiepin-4-ol.
C₃₄H₅₂N₂O₇S₁ (632.87) MS (ESI) 577 (M +2*H - tert.-Bu)

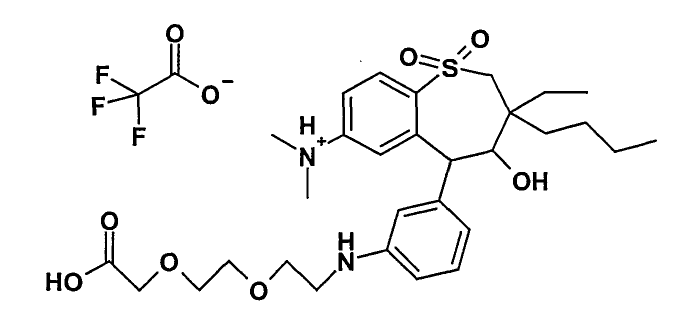

### d) (3-Butyl-5-{3-[2-(2-carboxymethoxy-ethoxy)-ethylamino]-phenyl}-3-ethyl-4-hydroxy-1,1- dioxo-2,3,4,5-tetrahydro-1H-benzo[b]thiepin-7-yl)-dimethyl-ammonium; trifluor- acetat (57)

Eine Lösung von 90 mg (2-{2-[3-(3-Butyl-7-dimethylamino-3-ethyl-4-hydroxy-1,1-dioxo-2,3,4,5-tetrahydro-1H-benzo[b]thiepin-5-yl)-phenylamino]-ethoxy}-ethoxy)-essigsäure -tert-butyl ester in 1 ml Methylenchlorid mit 1 ml Trifluoressigsäure wird 2 h gerührt und eingeengt. Das Produkt wird über HPLC (Knauer Eurospher-100-10-C18, Wasser (0.1 % Trifluoressigsäure)/Acetonitril (0.1% Trifluoressigsäure) = 80/20 -> 10/90) gereinigt.
C₃₀H₄₄N₂O₇S₁ (576.76) MS (ESI) 577 (M + H)

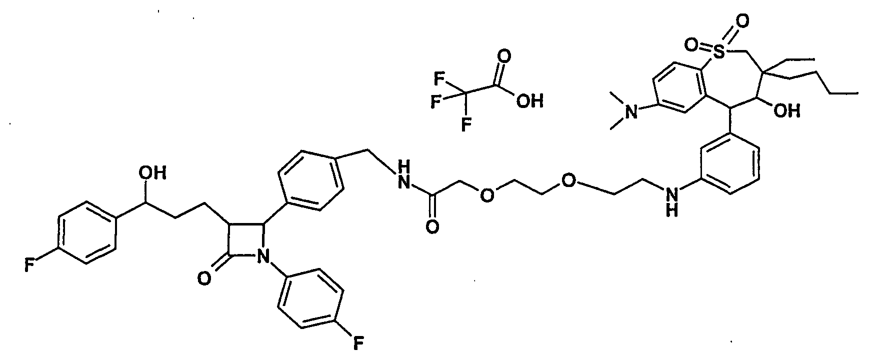

### e) {3-Butyl-3-ethyl-5-[3-(2-{2-[(4-{1-(4-fluor-phenyl)-3-[3-(4-fluor-phenyl)-3-hydroxy-propyl]-4-oxo- azetidin-2-yl}-benzylcarbamoyl)-methoxy]-ethoxy}-ethylamino)-phenyl]-4-hydroxy-1,1-dioxo- 2,3,4,5-tetrahydro-1H-benzo[b]thiepin-7-yl}-dimethyl-ammonium; trifluor- acetat (58)

Zu einer Lösung von 40 mg (2-{2-[3-(3-Butyl-7-dimethylamino-3-ethyl-4-hydroxy-1,1-dioxo-2,3,4,5-tetrahydro-1H-benzo[b]thiepin-5-yl)-phenylamino]-ethoxy}-ethoxy)-essigsäure-Verbindung mit Trifluoressigsäure, 37 µl Diisopropylcarbodiimid, 26 mg Hydroxybenzotriazol, 40 µl Triethylamin in 2 ml Dimethylformamid wird 55 mg 4-(4-Aminomethyl-phenyl)-1-(4-fluoro-phenyl)-3-[3-(4-fluoro-phenyl)-3-hydroxy-propyl]-azetidin-2-one und 12 h gerührt. Die Reaktionslöung wird eingeengt und über HPLC (Merck-Hibar-Lichrospher 100-RP-18, Wasser (0.1 % Trifluoressigsäure)/Acetonitril (0.1% Trifluoressigsäure) = 80/20 -> 10/90) getrennt.
C₅₇H₆₇F₅N₄O₁₀S₁ (1095.22) MS (ESI) 981 (M + H)

### Beispiel XXIX

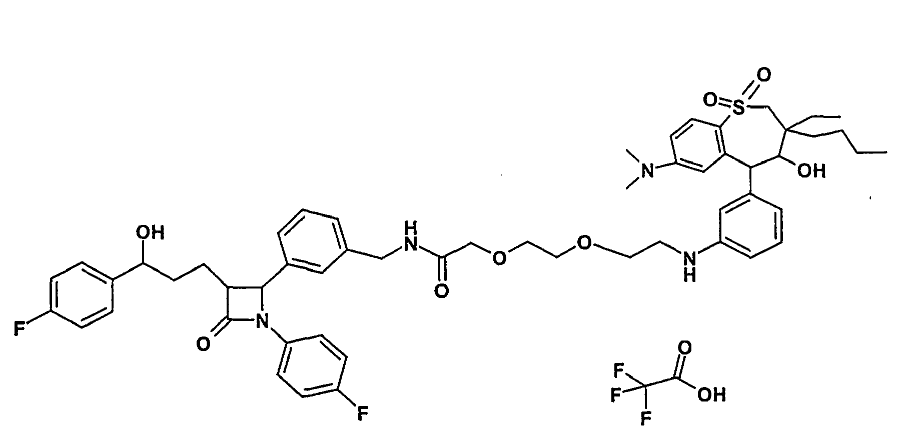

### {3-Butyl-3-ethyl-5-[3-(2-{2-[(3-{1-(4-Fluor-phenyl)-3-[3-(4-fluor-phenyl)-3-hydroxy-propyl]-4-oxo- azetidin-2-yl}-benzylcarbamoyl)-methoxy]-ethoxy}-ethylamino)-phenyl]-4-hydroxy-1,1-dioxo-2,3,4,5-tetrahydro-1H-benzo[b]thiepin-7-yl}-dimethyl-ammonium; trifluor- acetat (59)

Synthese analog 58.
C₅₇H₆₇F₅N₄O₁₀S₁ (1095.22) MS (ESI) 981 (M + H)

### Beispiel XXX

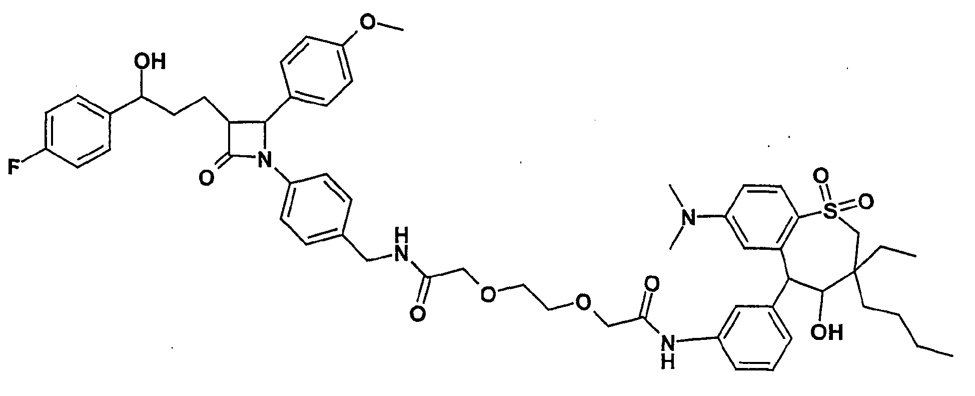

### 2-(2-{[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-methoxy}-ethoxy)-N-{4-[3-[3-(4-fluor-phenyl)-3-hydroxy-propyl]-2-(4-methoxy-phenyl)-4-oxo-azetidin-1-yl]-benzyl}-acetamid (65)

### a) 3-[5-(tert-Butyl-dimethyl-silanyloxy)-5-(4-fluor-phenyl)-pentanoyl]-4-phenyl-oxazolidin-2-on (60)

27 g 3-[5-(4-Fluor-phenyl)-5-hydroxy-pentanoyl]-4-phenyl-oxazolidin-2-on werden mit 13,6 g Tert.-Butyl-Dimethylsilylchlorid und 10,2 g Imidazol in 36 ml Dimethylformamid gelöst und 90 min. bei 60°C gerührt. Nach Beendigung der Reaktion wird das Gemisch in Essigsäureethylester gelöst und zweimal mit Wasser ausgeschüttelt. Die organische Phase wird über Magnesiumsulfat getrocknet, filtriert und im Vakuum eingeengt. Man erhält 3-[5-(tert-Butyl-dimethyl-silanyloxy)-5-(4-fluor-phenyl)-pentanoyl]-4-phenyl- oxazolidin-2-on mit dem Molekulargewicht 471,65 (C₂₆H₃₄FNO₄Si); MS (ESI): 340.28 (MH⁺-HOSi(CH₃)₂C(CH₃)₃)

### b) 4-[5-(tert-Butyl-dimethyl-silanyloxy)-5-(4-fluor-phenyl)-1-(4-methoxy-phenyl)-2-(2-oxo- 4-phenyl-oxazolidin-3-carbonyl)-pentylamino]-benzonitril (61)

16,2 g 3-[5-(tert-Butyl-dimethyl-silanyloxy)-5-(4-fluor-phenyl)-pentanoyl]-4-phenyl-oxazolidin-2-on werden in 350 ml Dichlormethan gelöst. Die Lösung wird mit 19,8 ml Hünig Base und mit 10,14 g 4-[(4-Methoxy-phenylimino)-methyl]-benzonitril versetzt und auf -10°C gekühlt. Zur gekühlten Lösung fügt man 8,52 ml Trimethylsilyltriflat hinzu und rührt 30 min. bei -10°C. Die Lösung wird nun auf -30°C abgekühlt, und es werden 44 ml Titantetrachloridlösung zugegeben. Die Reaktionsmischung wird 2 h bei -30 bis-40°C gerührt. Danach lässt man die Lösung sich auf Raumtemperatur erwärmen, wäscht die Reaktionslösung nacheinander mit 200 ml 2N Schwefelsäure, 300 ml 20%iger Natriumhydrogensulfitlösung und ges. Kochsalzlösung. Die organische Phase wird über Magnesiumsulfat getrocknet, im Vakuum eingeengt und der Rückstand wird über Kieselgel mit n-Heptan/Essigsäureethylester 3/1 gereinigt. Man erhält 4-[5-(tert-Butyl-dimethyl-silanyloxy)-5-(4-fluor-phenyl)-1-(4-methoxy-phenyl)-2-(2-oxo- 4-phenyl-oxazolidin-3-carbonyl)-pentylamino]-benzonitril mit dem Molekulargewicht 707,93 (C₄₁H₄₆FN₃O₅Si); MS (ESI): 590.51 (MH⁺- C₇H₅N₂).

### c) 4-[3-[3-(tert-Butyl-dimethyl-silanyloxy)-3-(4-fluor-phenyl)-propyl]-2-(4-methoxy-phenyl)-4-oxo-azetidin-1-yl]-benzonitril (62)

13,2 g 4-[5-(tert-Butyl-dimethyl-silanyloxy)-5-(4-fluor-phenyl)-1-(4-methoxy-phenyl)-2-(2-oxo- 4-phenyl-oxazolidin-3-carbonyl)-pentylamino]-benzonitril werden in 380 ml Methyl-tert.-Butylether gelöst, mit 18,6 ml N,O-Bis(trimethylsilyl)-acetamid und 1,86 ml einer 1 M Lösung von Tetrabutylammoniumfluorid in Tetrahydrofuran versetzt und 2 h bei Raumtemperatur gerührt. Nach Beendigung der Reaktion fügt man 10 ml Essigsäure zu, engt die Reaktionsmischung im Vakuum ein und reinigt den Rückstand über Kieselgel mit Toluol/Essigsäureethylester 50/1. Man erhält 4-[3-[3-(tert-Butyl-dimethyl-silanyloxy)-3-(4-fluor-phenyl)-propyl]-2-(4-methoxy- phenyl)-4-oxo-azetidin-1-yl]-benzonitril mit dem Molekulargewicht 544,75 (C₃₂H₃₇FN₂O₃Si); MS (ESI): 545.56 (M+H⁺).

### d) 4-[3-[3-(4-Fluor-phenyl)-3-hydroxy-propyl]-2-(4-methoxy-phenyl)-4-oxo-azetidin-1-yl]- benzonitril (63)

3.5 g 4-[3-[3-(tert-Butyl-dimethyl-silanyloxy)-3-(4-fluor-phenyl)-propyl]-2-(4-methoxy-phenyl)-4-oxo-azetidin-1-yl]-benzonitril werden in 65 ml Tetrahydrofuran gelöst, mit 0,74 ml Essigsäure und 8,03 ml einer 1 M Lösung von Tetrabutylammoniumfluorid in Tetrahydrofuran versetzt und 2 h bei Raumtemperatur gerührt. Danach werden 4,82 ml der Tetrabutylammoniumfluorid-Lösung nachgegeben und weitere 3 h bei Rückflusstemperatur gerührt. Die abgekühlte Reaktionsmischung wird im Vakuum eingeengt, und der Rückstand wird chromatographisch über Kieselgel mit n-Heptan/Essigsäureethylester 2/1 gereinigt. Man erhält 4-[3-[3-(4-Fluor-phenyl)-3-hydroxy-propyl]-2-(4-methoxy-phenyl)-4-oxo-azetidin-1-yl]- benzonitril mit dem Molekulargewicht 430,48 (C₂₆H₂₃FN₂O₃); MS (ESI): 431.24 (M+H⁺).

### e) 1-(4-Aminomethyl-phenyl)-3-[3-(4-fluor-phenyl)-3-hydroxy-propyl]-4-(4-methoxy-phenyl)- azetidin-2-on (64)

1,22 g 4-[3-[3-(4-Fluor-phenyl)-3-hydroxy-propyl]-2-(4-methoxy-phenyl)-4-oxo-azetidin-1-yl]- benzonitril werden in 90 ml Ethanol gelöst, mit 10 ml konz. Ammoniaklösung und einem Überschuß Raney-Nickel versetzt und 8 h bei 60°C und einem Druck von 10 bar Wasserstoff gerührt. Die Reaktionsmischung kühlt über Nacht auf Raumtemperatur ab; anderntags wird vom Katalysator abgetrennt, das Filtrat im Vakuum eingeengt und der Rückstand chromatographisch über Kieselgel mit Dichlormethan/Methanol/Ammoniak-Lösung 10/1/0.1 gereinigt. Man erhält 1-(4-Aminomethyl-phenyl)-3-[3-(4-fluor-phenyl)-3-hydroxy-propyl]-4-(4-methoxy-phenyl)-azetidin-2-on mit dem Molekulargewicht 434,51 (C₂₆H₂₇FN₂O₃); MS (ESI): 418.2 (MH⁺- NH₃).

### f) 2-(2-{[3-(3-Butyl-7-dimethylamino-3-ethyl-4-hydroxy-1,1-dioxo-2,3,4,5-tetrahydro-1H- benzo[b]thiepin-5-yl)-phenylcarbamoyl]-methoxy}-ethoxy)-N-{4-[3-[3-(4-fluor-phenyl)- 3-hydroxy-propyl]-2-(4-methoxy-phenyl)-4-oxo-azetidin-1-yl]-benzyl}-acetamid (65)

140 mg (2-{[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-methoxy}-ethoxy)-essigsäure (17) und 100 mg 1-(4-Aminomethyl-phenyl)-3-[3-(4-fluor-phenyl)-3-hydroxy-propyl]-4-(4-methoxy-phenyl)- azetidin-2-on werden bei Raumtemperatur in 5 ml Dimethylformamid gelöst, mit 35 mg 1-Hydroxy-benzotriazol und 45 mg 1-Ethyl-3-(3-dimethylaminopropyl)carbodiimid Hydrochlorid versetzt und 6 h bei Raumtemperatur gerührt. Die Reaktionsmischung wird im Vakuum eingeengt. Der Rückstand wird mit Dichlormethan versetzt, zweimal mit Wasser und einmal mit ges. Kochsalzlösung ausgeschüttelt; der organische Extrakt wird über Magnesiumsulfat getrocknet, filtriert und im Vakuum eingeengt. Das Rohprodukt wird chromatographisch (RP18; Dichlormethan/Methanol 96/4 nach Dichlormethan/Methanol 92/8 innerhalb 25 min.) gereinigt. Man erhält das Produkt mit dem Schmelzpunkt 116-125°C. Molekulargewicht 1007,24 (C₅₆H₆₇FN₄O₁₀S); MS (ESI): 1008.53 (M+H⁺).

### Beispiel XXXI

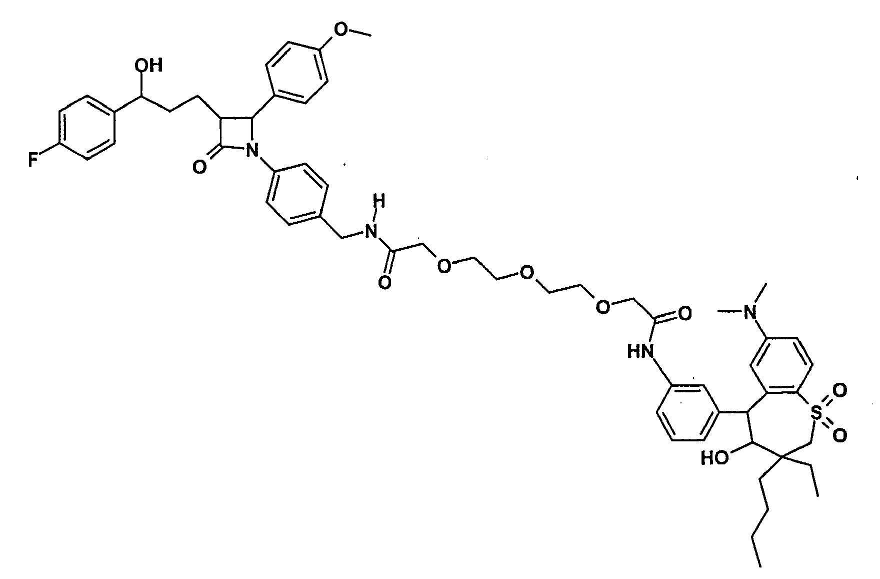

### N-[3-(3-Butyl-7-dimethylamino-3-ethyl-4-hydroxy-1,1-dioxo-2,3,4,5-tetrahydro-1H-benzo[b]thiepin-5- yl)-phenyl]-2-{2-[2-({4-[3-[3-(4-fluoro-phenyl)-3-hydroxy-propyl]-2-(4-methoxy-phenyl)-4- oxo-azetidin-1-yl]-benzylcarbamoyl}-methoxy)-ethoxy]-ethoxy}-acetamid (66)

Die Verbindung des Beispiels 3 wird wie die des Beispiels 2 hergestellt, mit dem Unterschied, dass statt (17) [2-(2-{[3-(3-Butyl-7-dimethylamino-3-ethyl-4-hydroxy-1,1-dioxo-2,3,4,5-tetrahydro-1H-benzo[b]thiepin-5-yl)-phenylcarbamoyl]-methoxy}-ethoxy)-ethoxy]-essigsäure (20) eingesetzt wurde.
Molekulargewicht 1051,29 (C₅₈H₇₁FN₄O₁₁S); MS (ESI): 1052.51 (M+H⁺).

Die erfindungsgemäßen Verbindungen der Formel I wurden mit der nachfolgend beschriebenen Methode auf ihre Wirkung geprüft:

### Beeinflussung der Cholesterolabsorption + ³H- Taurocholsäureausscheidung anhand der fäkalen Ausscheidung an der Maus, Ratte oder Hamster

NMRI- Mäuse, Wistar-Ratten, oder Golden Syrian Hamster (in Gruppen von n=4-6) werden unter Standarddiät (Altromin, Lage (Lippe)) in Stoffwechselkäfigen gehalten. Am Nachmittag vor Gabe der radioaktiven Tracer (¹⁴C-Cholesterol) werden die Tiere nüchtern gesetzt und auf Gitterroste adaptiert.

Zusätzlich werden die Tiere werden 24 Stunden vor der peroralen Applikation der Testmahlzeit (¹⁴C-Cholesterol in Intralipid® 20, Pharmacia-Upjohn) mit ³H-TCA (Taurocholic acid) s.c. gelabelt (z.b. 1 µCi/Maus bis 5 µCi/Ratte)

Cholesterolabsorptionstest: 0,25 ml/Maus Intralipid® 20 (Pharmacia- Upjohn) (Spikung mit 0,25 µCi ¹⁴C-Cholesterol in 0,1 mg Cholesterol) werden peroral mit der Schlundsonde verabreicht.

Testsubstanzen werden getrennt in 0,5 % Methylcellulose (Sigma)/5% Solutol (BASF, Ludwigshafen) oder geeignetem Vehikel angesetzt.
Das Applikationsvolumen der Testsubstanz beträgt 0,5 ml/Maus. Die Testsubstanz wird unmittelbar vor der Testmahlzeit (intralipid mit ¹⁴C-Cholesterol-label) (Cholesterolabsorptionstest) appliziert.

Der Kot wird über 24 h gesammelt: die fäkale Elimination von ¹⁴C-Cholesterol und ³H Taurocholsäure (TCA) nach 24 Std. wird bestimmt.

Die Lebern werden entnommen, homogenisiert und Aliquots im Oximaten (Model 307, Packard) verbrannt zur Bestimmung der aufgenommenn/resorbierten Menge an ¹⁴C- Cholesterol.

### Auswertung:

### Kotproben:

Gesamtgewicht bestimmen, mit Wasser auf definiertes Volumen auffüllen, dann homogenisieren, Aliquot eintrockenen und im Oximat (Model 307, Packard zur Verbrennung von radioaktiv gelabelten Proben) verbrennen: Die Menge von radioaktiv ³H- H₂O und ¹⁴C- CO₂ wird hochgerechnet auf die ausgeschiedene Menge an ³H-Taurocholsäure bzw. ¹⁴C-Cholesterol (Dual-Isotopen-Technik ). Die ED₂₀₀₋Werte werden als Dosis aus einer Dosiswirkungskurve interpoliert als diejenige Dosen, die die Auscheidung an TCA bzw. Cholesterol verdoppeln, bezogen auf eine zeitgleich behandelte Kontrollgruppe.

### Leberproben:

Die aufgenommene Menge von ¹⁴C-Cholesterols in die Leber wird bezogen auf die applizierte Dosis. Die ED₅₀ Werte werden interpoliert aus einer Dosiswirkungskurve als diejenige Dosis, die die Aufnahme von ¹⁴C- Cholesterol in die Leber halbiert (50%), bezogen auf eine Kontrollgruppe

Die folgenden ED₅₀-Werte belegen die Aktivität der erfindungsgemäßen Verbindungen der Formel I

| **Beispiel Nr.** | **ED**_{**50**} **(Leber) [mg/Maus]** |
|---|---|
| II | 0.01 |
| III | 0.03 |
| VIII | 0.003 |
| XXV | 0.01 |
| XXXI | 0.1 |

Aus der Tabelle ist abzulesen, daß die Verbindungen der Formel I eine sehr gute Cholesterin senkende Wirkung besitzen.

### Resorbierbarkeit:

Die Resorbierbarkeit der Verbindungen der Formel I wurde Caco-Zellmodell geprüft (A.R. Hilgers et al., Caco-2 cell monolayers as a model for drug transport across the intestinal mucosa, Pharm. Res. 1990 , 7, 902).

Aus den Meßdaten ist abzulesen, daß die erfindungsgemäßen Verbindungen der Formel I gegenüber den im Stand der Technik beschriebenen Verbindungen (Referenzstruktur) eine deutlich geringere Resorption aufweisen:

| | Referenzstruktur | Beispiel XII |
|---|---|---|
| Apparenter Partitionskoeffizient Pₐₚₚ [cm/s] (entsprechend Lit. Hilgers) | 4.88 x 10⁻⁰⁶ | 3.67 x 10⁻⁰⁹ |
| Abgeschätzte Human-Resorption | 100% | < 1% |

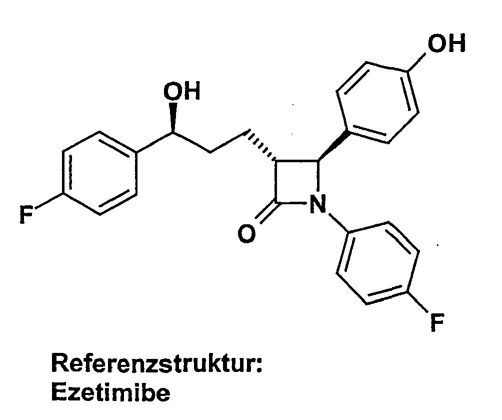

## Patentansprüche

1. Verbindungen der Formel I, 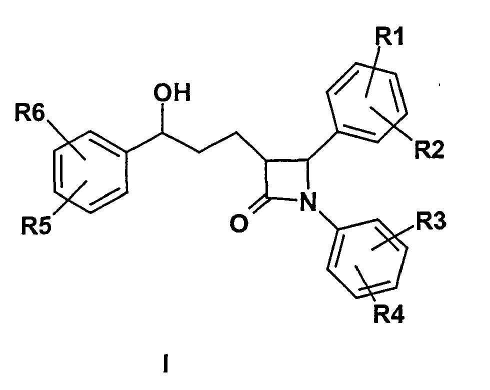 worin bedeuten
R1, R2, R3, R4, R5, R6 unabhängig voneinander (C₀-C₃₀)-Alkylen-L, wobei ein oder mehrere C-Atome des Alkylenrests durch -O-, -(C=O)-, -CH=CH-, -C≡C-, -N((C₁-C₆)-Alkyl)- oder -NH- ersetzt sein können;
H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
L 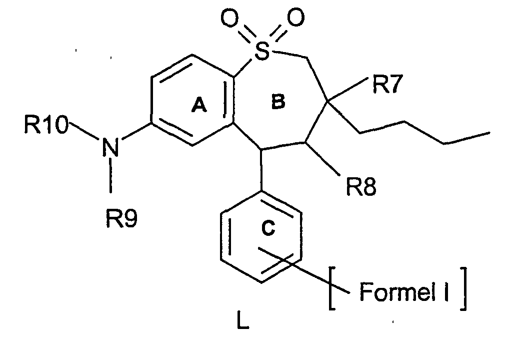
R7 Methyl, Ethyl, Propyl, Butyl;
R8 H, OH, NH₂, NH-(C₁-C₆)-Alkyl;
R9 Methyl, Ethyl, Propyl, Butyl;
R10 Methyl, Ethyl, Propyl, Butyl;
wobei immer mindestens einer der Reste R1 bis R6 die Bedeutung (C₀-C₃₀)-Alkylen-L, wobei ein oder mehrere C-Atome des Alkylenrests durch -O-, -(C=O)-, -CH=CH-, -C≡C-, -N((C₁-C₆)-Alkyl)- oder -NH- ersetzt sein können, besitzen muß,
sowie deren pharmazeutisch verträglichen Salze.

2. Verbindungen der Formel I, gemäß Anspruch 1, **dadurch gekennzeichnet, daß** darin bedeuten
R1, R2, R3, R4, R5, R6 unabhängig voneinander (C₀-C₃₀)-Alkylen-L, wobei ein oder mehrere C-Atome des Alkylenrests durch -O-, -(C=O)-oder -NH- ersetzt sein können;
H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
L 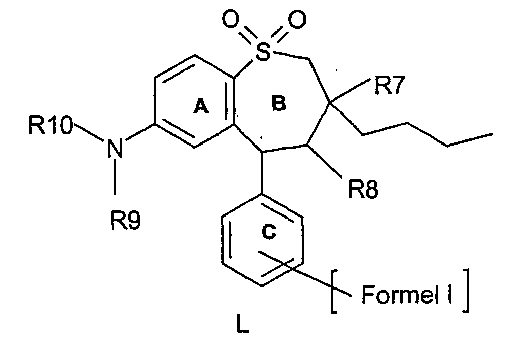
R7 Methyl, Ethyl, Propyl, Butyl;
R8 H, OH, NH₂, NH-(C₁-C₆)-Alkyl;
R9 Methyl, Ethyl, Propyl, Butyl;
R10 Methyl, Ethyl, Propyl, Butyl;
wobei immer mindestens einer der Reste R1 bis R6 die Bedeutung (C₀-C₃₀)-Alkylen-L, wobei ein oder mehrere C-Atome des Alkylenrests durch -O-, -(C=O)- oder -NH- ersetzt sein können, besitzt,
sowie deren pharmazeutisch verträglichen Salze.

3. Verbindungen der Formel I, gemäß Anspruch 1 oder 2, **dadurch gekennzeichnet, daß** darin bedeuten
R1, R2, R3, R4, R5, R6 unabhängig voneinander (C₀-C₃₀)-Alkylen-L, wobei ein oder mehrere C-Atome des Alkylenrests durch -O-, -(C=O)- oder -NH- ersetzt sein können;
H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
L 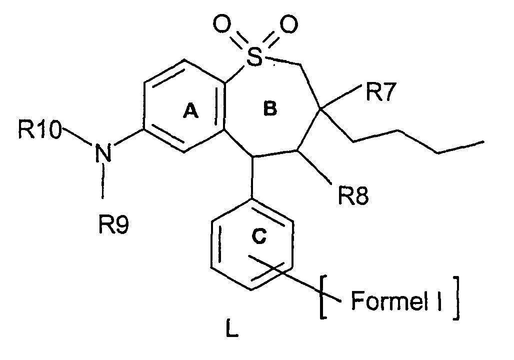
R7 Methyl, Ethyl, Propyl, Butyl;
R8 H, OH, NH₂, NH-(C₁-C₆)-Alkyl;
R9 Methyl, Ethyl, Propyl, Butyl;
R10 Methyl, Ethyl, Propyl, Butyl;
wobei einer der Reste R1 oder R3 die Bedeutung (C₀-C₃₀)-Alkylen-L, wobei ein oder mehrere C-Atome des Alkylenrests durch -O-, -(C=O)- oder -NH- ersetzt sein können, besitzt,
sowie deren pharmazeutisch verträglichen Salze.

4. Verbindungen der Formel I gemäß einem oder mehreren der Ansprüche 1 bis 3, **dadurch gekennzeichnet, daß** darin bedeuten
R1, R2, R3, R4, R5, R6 unabhängig voneinander -(CH₂)₀₋₁-NH-(C=O)₀₋₁-(C₃-C₂₅)-Alkylen-(C=O)₀₋₁-NH-L ,wobei ein oder mehrere C-Atome des Alkylenrests durch O-Atome ersetzt sein können;
H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
L 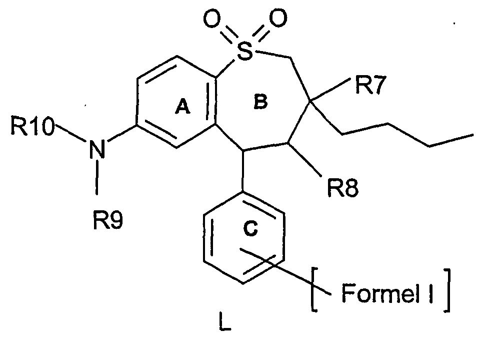
R7 Methyl, Ethyl, Propyl, Butyl;
R8 H, OH, NH₂, NH-(C₁-C₆)-Alkyl;
R9 Methyl, Ethyl, Propyl, Butyl;
R10 Methyl, Ethyl, Propyl, Butyl;
worin einer der Reste R1 oder R3 die Bedeutung -(CH₂)₀₋₁-NH-(C=O)₀₋₁-(C₃-C₂₅)-Alkylen-(C=O)₀₋₁-NH-L ,wobei ein oder mehrere C-Atome des Alkylenrests durch O-Atome ersetzt sein können, besitzt,
sowie deren pharmazeutisch verträglichen Salze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und mindestens einen weiteren Wirkstoff.

7. Arzneimittel, gemäß Anspruch 6, **dadurch gekennzeichnet, daß** es als weiteren Wirkstoff eine oder mehrere Verbindungen, die den Lipidstoffwechsel normalisieren, enthält.

8. Arzneimittel, gemäß Anspruch 6 oder 7, **dadurch gekennzeichnet, daß** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyte-stimulierendes Hormon)-Agonisten, CCK-Agonisten, Serotonin-Wiederaufnahme-Inhibitoren, gemischte Serotonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptine, Doprexin), Lipase/Amylase-Inhibitoren, PPAR-Modulatoren, RXR-Modulatoren oder TR-β-Agonisten oder Amphetamine enthält.

9. Verbindungen gemäß einem oder mehreren der Ansprüche 1 bis 4 zur Anwendung als Medikament zur Behandlung von Lipidstoffwechselstörungen.

10. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

11. Verwendung der Verbindungen gemäß einem oder mehreren der Ansprüche 1 bis 4 zur Herstellung eines Medikaments zur Behandlung von Hyperlipidämie.

12. Verwendung der Verbindungen gemäß einem oder mehreren der Ansprüche 1 bis 4 zur Herstellung eines Medikaments zur Senkung des Serumcholesterinspiegels.

13. Verwendung der Verbindungen gemäß einem oder mehreren der Ansprüche 1 bis 4 zur Herstellung eines Medikaments zur Behandlung arteriosklerotischer Erscheinungen.

14. Verwendung der Verbindungen gemäß einem oder mehreren der Ansprüche 1 bis 4 zur Herstellung eines Medikaments zur Behandlung von Insulin Resistenz.

## Claims

1. A compound of the formula I, 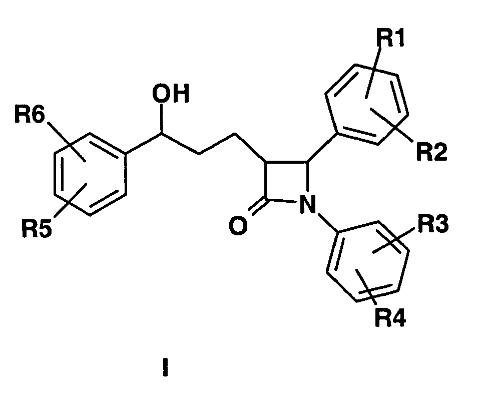 in which
R1, R2, R3, R4, R5, R6 independently of one another are (C₀-C₃₀)-alkylene-L, where one or more carbon atoms of the alkylene radical may be replaced by -O-, -(C=O)-, -CH=CH-, -C≡C-, -N((C₁-C₆)-alkyl)- or -NH-;
H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more or all hydrogens in the alkylene radicals may be replaced by fluorine; SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
is
L 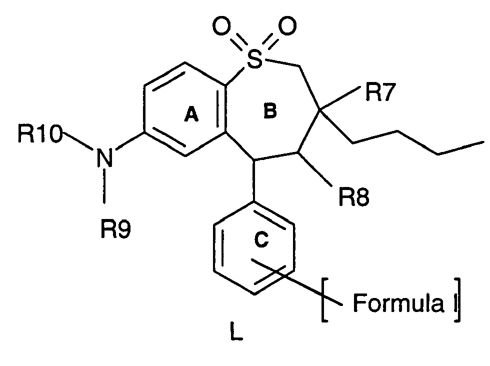
R7 is methyl, ethyl, propyl, butyl;
R8 is H, OH, NH₂, NH-(C₁-C₆)-alkyl;
R9 is methyl, ethyl, propyl, butyl;
R10 is methyl, ethyl, propyl, butyl;
where in each case at least one of the radicals R1 to R6 must have the meaning (C₀-C₃₀)-alkylene-L, where one or more carbon atoms of the alkylene radical may be replaced by -O-, -(C=O)-, -CH=CH-, -C≡C-, -N((C₁-C₆)-alkyl)- or -NH-,
and its pharmaceutically acceptable salts.

2. A compound of the formula I, as claimed in claim 1, wherein
R1, R2, R3, R4, R5, R6 independently of one another are (C₀-C₃₀)-alkylene-L, where one or more carbon atoms of the alkylene radical may be replaced by -O-, -(C=O)- or -NH-;
H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more or all hydrogens in the alkylene radicals may be replaced by fluorine;
SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
is
L 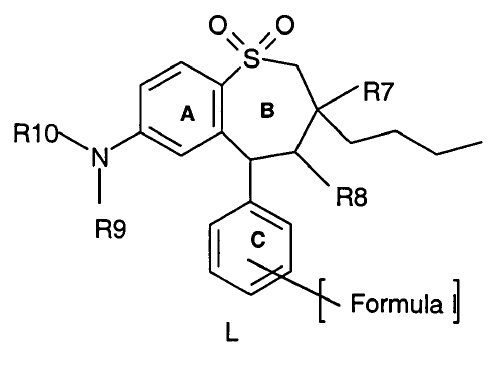
R7 is methyl, ethyl, propyl, butyl;
R8 is H, OH, NH₂, NH-(C₁-C₆)-alkyl;
R9 is methyl, ethyl, propyl, butyl;
R10 is methyl, ethyl, propyl, butyl;
where in each case at least one of the radicals R1 to R6 has the meaning (C₀-C₃₀)-alkylene-L, where one or more carbon atoms of the alkylene radical may be replaced by -O-, -(C=O)- or -NH-,
and its pharmaceutically acceptable salts.

3. A compound of the formula I, as claimed in claim 1 or 2, wherein
R1, R2, R3, R4, R5, R6 independently of one another are (C₀-C₃₀)-alkylene-L, where one or more carbon atoms of the alkylene radical may be replaced by -O-, -(C=O)- or -NH-;
H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more or all hydrogens in the alkylene radicals may be replaced by fluorine;
SO₂-NH₂, SO₂NH(C₁-C₆)-alkyl, SO₂N[(C₁-C₆)-alkyl]₂ , S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
is
L 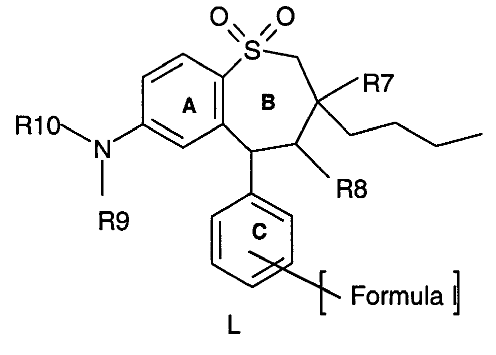
R7 is methyl, ethyl, propyl, butyl;
R8 is H, OH, NH₂, NH-(C₁-C₆)-alkyl;
R9 is methyl, ethyl, propyl, butyl;
R10 is methyl, ethyl, propyl, butyl;
where one of the radicals R1 or R3 has the meaning (C₀-C₃₀)-alkylene-L, where one or more carbon atoms of the alkylene radical may be replaced by -O-, -(C=O)- or -NH-,
and its pharmaceutically acceptable salts.

4. A compound of the formula I as claimed in one or more of claims 1 to 3, wherein
R1, R2, R3, R4, R5, R6 independently of one another are -(CH₂)₀₋₁-NH-(C=O)₀₋₁-(C₃-C₂₅)-alkylene-(C=O)₀₋₁-NH-L, where one or more carbon atoms of the alkylene radical may be replaced by oxygen atoms;
H, F, Cl, Br, I, CF₃, NO₂, CN, COOH, COO(C₁-C₆)alkyl, CONH₂, CONH(C₁-C₆)alkyl, CON[(C₁-C₆)alkyl]₂, (C₁-C₆)-alkyl, (C₂-C₆)-alkenyl, (C₂-C₆)-alkynyl, O-(C₁-C₆)-alkyl, where one, more or all hydrogens in the alkylene radicals may be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
is
L 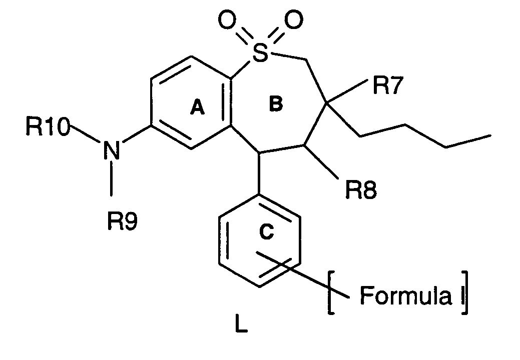
R7 is methyl, ethyl, propyl, butyl;
R8 is H, OH, NH₂, NH-(C₁-C₆)-alkyl;
R9 is methyl, ethyl, propyl, butyl;
R10 is methyl, ethyl, propyl, butyl;
where one of the radicals R1 to R3 has the meaning -(CH₂)₀₋₁-NH-(C=O)₀₋₁-(C₃-C₂₅)-alkylene-(C=O)₀₋₁-NH-L, where one or more carbon atoms of the alkylene radical may be replaced by oxygen atoms,
and its pharmaceutically acceptable salts.

5. A medicament comprising one or more of the compounds as claimed in one or more of claims 1 to 4.

6. A medicament comprising one or more of the compounds as claimed in one or more of claims 1 to 4 and at least one further active compound.

7. The medicament as claimed in claim 6, comprising, as further active compound, one or more compounds which normalize lipid metabolism.

8. The medicament as claimed in claim 6 or 7, which comprises, as further active compound, one or more antidiabetics, hypoglycemically active compounds, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyases inhibitors, squalene synthetase inhibitors, lipoprotein(a) antagonists, lipase inhibitors, insulins, sulphonyl ureas, biguanides, meglitinides, thiolidindiones, α-glucosidase inhibitors, active compounds which act on the ATP-dependent potassium channel of the beta cells, CART agonists, NPY agonists, MC4 agonists, orexin agonists, H3 agonists, TNF agonists, CRF agonists, CRF BP agonists, urocortin agonists, β3 agonists, MSH (melanocyte-stimulating hormone) agonists, CCK agonists, serotonin-reuptake inhibitors, mixed serotonin and noradrenergic compounds, 5HT agonists, bombesin agonists, galanin agonists, growth hormones, growth hormone-releasing compounds, TRH agonists, decoupling protein 2- or 3-modulators, leptin agonists, DA agonists (bromocriptine, doprexin), lipase/amylase inhibitors, PPAR modulators, RXR modulators or TR-β-agonists or amphetamines.

9. A compound as claimed in one or more of claims 1 to 4 for use as a medicament for the treatment of impaired lipid metabolism.

10. A process for preparing a medicament comprising one or more of the compounds as claimed in one or more of claims 1 to 4, which comprises mixing the active compound with a pharmaceutically acceptable carrier and bringing this mixture into a form suitable for administration.

11. The use of the compounds as claimed in one or more of claims 1 to 4 for preparing a medicament for treating hyperlipidemia.

12. The use of the compounds as claimed in one or more of claims 1 to 4 for preparing a medicament for lowering the serum cholesterol concentration.

13. The use of the compounds as claimed in one or more of claims 1 to 4 for preparing a medicament for treating arteriosclerotic symptoms.

14. The use of the compounds as claimed in one or more of claims 1 to 4 for preparing a medicament for treating insulin resistance.

## Revendications

1. Composés de formule I 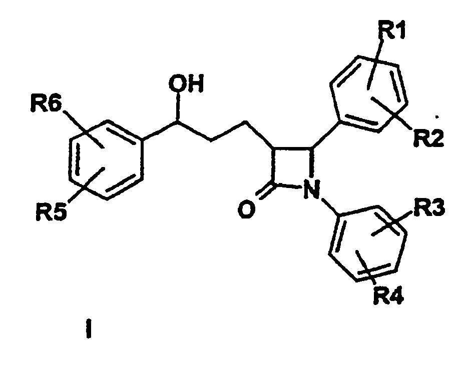 dans laquelle
R1, R2, R3, R4, R5, R6 représentent, indépendamment les uns des autres, un groupe alkylène(C₀-C₃₀)-L, un ou plusieurs atomes de carbone du radical alkylène pouvant être remplacés par -O-, -(C=O)-, -CH=CH-, -C≡C-, -N[alkyle(C₁-C₆)- ou -NH- ;
H, F, Cl, Br, I, CF₃, NO₂, CN, COOH, COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle(C₁-C₆), dans les radicaux alkyle un, plusieurs ou tous les atome(s) d'hydrogène pouvant être remplacé(s) par le fluor ; SO₂-NH₂, SO₂NH-alkyle(C₁-C₆), SO₂N[alkyle(C₁-C₆)]₂, S-alkyle(C₁-C₆), S-(CH₂)ₙ-phényle, SO-alkyle(C₁-C₆), SO-(CH₂)ₙ-phényle, SO₂-alkyle(C₁-C₆), SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle(C₁-C₆), alkyle en C₁-C₆, NH₂ ;
NH₂, NH-alkyle(C₁-C₆), N-[alkyle(C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle(C₁-C₆), alkyle en C₁-C₆, NH₂, NH-alkyle(C₁-C₆), N[alkyle(C₁-C₆)]₂, SO₂CH₃, COOH, COO-alkyle(C₁-C₆), CONH₂ ;
L représente 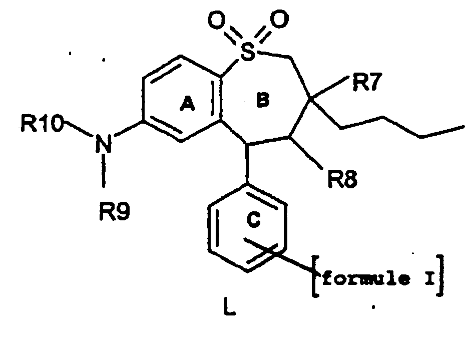
R7 représente le groupe méthyle, éthyle, propyle, butyle ;
R8 représente H, OH, NH₂, un groupe NH-alkyle(C₁-C₆) ;
R9 représente le groupe méthyle, éthyle, propyle, butyle ;
R10 représente le groupe méthyle, éthyle, propyle, butyle ;
au moins un des radicaux R1 à R6 devant toujours avoir la signification d'un groupe alkylène (C₀-C₃₀) -L, un ou plusieurs atomes de carbone du radical alkylène pouvant être remplacés par -O-, -(C=O)-, -CH=CH-, -C≡C-, -N[alkyle(C₁-C₆)- ou -NH-,
ainsi que leurs sels pharmaceutiquement acceptables.

2. Composés de formule I, selon la revendication 1, **caractérisés en ce que** dans cette formule
R1, R2, R3, R4, R5, R6 représentent, indépendamment les uns des autres, un groupe alkylène(C₀-C₃₀)-L, un ou plusieurs atomes de carbone du radical alkylène pouvant être remplacés par -O-, -(C=O)- ou -NH- ;
H, F, Cl, Br, I, CF₃, NO₂, CN, COOH, COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle(C₁-C₆), dans les radicaux alkyle un, plusieurs ou tous les atome(s) d'hydrogène pouvant être remplacé(s) par le fluor ;
SO₂-NH₂, SO₂NH-alkyle(C₁-C₆), SO₂N[alkyle (C₁-C₆)]₂, S-alkyle(C₁-C₆), S-(CH₂)ₙ-phényle, SO-alkyle(C₁-C₆), SO-(CH₂)ₙ-phényle, SO₂-alkyle(C₁-C₆), SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle(C₁-C₆), alkyle en C₁-C₆, NH₂ ;
NH₂, NH-alkyle (C₁-C₆), N-[alkyle(C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle(C₁-C₆), alkyle en C₁-C₆, NH₂, NH-alkyle(C₁-C₆), N[alkyle(C₁-C₆)]₂, SO₂CH₃, COOH, COO-alkyle(C₁-C₆), CONH₂ ;
L représente 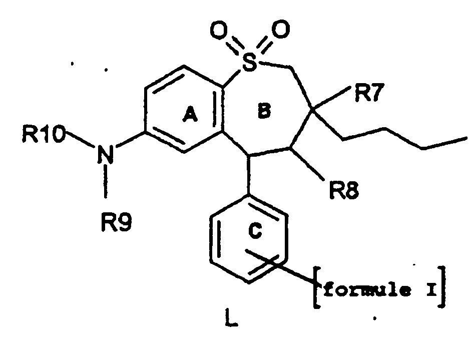
R7 représente le groupe méthyle, éthyle, propyle, butyle ;
R8 représente H, OH, NH₂, un groupe NH-alkyle(C₁-C₆);
R9 représente le groupe méthyle, éthyle, propyle, butyle ;
R10 représente le groupe méthyle, éthyle, propyle, butyle ;
au moins un des radicaux R1 à R6 devant toujours avoir la signification d'un groupe alkylène(C₀-C₃₀)-L, un ou plusieurs atomes de carbone du radical alkylène pouvant être remplacés par -O-, -(C=O)- ou -NH-,
ainsi que leurs sels pharmaceutiquement acceptables.

3. Composés de formule I, selon la revendication 1 ou 2, **caractérisés en ce que** dans cette formule
R1, R2, R3, R4, R5, R6 représentent, indépendamment les uns des autres, un groupe alkylène(C₀-C₃₀)-L, un ou plusieurs atomes de carbone du radical alkylène pouvant être remplacés par -O-, -(C=O)- ou -NH- ;
H, F, Cl, Br, I, CF₃, NO₂, CN, COOH, COO-alkyle(C₁-C₆), CONH₂, CONH-alkyle(C₁-C₆), CON[alkyle(C₁-C₆)]₂, alkyle en C₁-C₆, alcényle en C₂-C₆, alcynyle en C₂-C₆, O-alkyle (C₁-C₆), dans les radicaux alkyle un, plusieurs ou tous les atome(s) d'hydrogène pouvant être remplacé (s) par le fluor ;
SO₂-NH₂, SO₂NH-alkyle(C₁-C₆), SO₂N[alkyle (C₁-C₆)]₂, S-alkyle(C₁-C₆), S-(CH₂)ₙ-phényle, SO-alkyle(C₁-C₆), SO-(CH₂)ₙ-phényle, SO₂-alkyle(C₁-C₆), SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle(C₁-C₆), alkyle en C₁-C₆, NH₂ ;
NH₂, NH-alkyle(C₁-C₆), N-[alkyle(C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle(C₁-C₆), alkyle en C₁-C₆, NH₂, NH-alkyle(C₁-C₆), N[alkyle(C₁-C₆)]₂, SO₂CH₃, COOH, COO-alkyle(C₁-C₆), CONH₂ ;
L représente 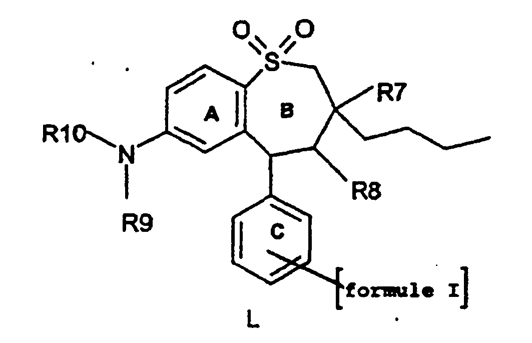
R7 représente le groupe méthyle, éthyle, propyle, butyle ;
R8 représente H, OH, NH₂, un groupe NH-alkyle(C₁-C₆) ;
R9 représente le groupe méthyle, éthyle, propyle, butyle ;
R10 représente le groupe méthyle, éthyle, propyle, butyle ;
l'un des radicaux R1 et R3 ayant la signification d'un groupe alkylène(C₀-C₃₀)-L, un ou plusieurs atomes de carbone du radical alkylène pouvant être remplacés par -O-, -(C=O)- ou -NH-,
ainsi que leurs sels pharmaceutiquement acceptables.

4. Composés de formule I, selon une ou plusieurs des revendications 1 à 3, **caractérisés en ce que** dans cette formule
R1, R2, R3, R4, R5, R6 représentent, indépendamment les uns des autres, un groupement -CH₂)₀₋₁-NH-(C=O)₀₋₁-alkylène(C₃-C₂₅)-(C=O)₀₋₁-NH-L, un ou plusieurs atomes de carbone du radical alkylène pouvant être remplacés par des atomes d'oxygène ;
H, F, Cl, Br, I, CF₃, NO₂, CN, COOH, COO-alkyle (C₁-C₆), CONH₂, CONH-alkyle(C₁-C₆), CON [alkyle (C₁-C₆)]₂, alkyle en C₁-C₆, alcényle en C₂-C₆, alcynyle en C₂-C₆, O-alkyle(C₁-C₆), dans les radicaux alkyle un, plusieurs ou tous les atome(s) d'hydrogène pouvant être remplacé(s) par le fluor ;
SO₂-NH₂, SO₂NH-alkyle(C₁-C₆), SO₂N [alkyle (C₁-C₆)]₂, S-alkyle(C₁-C₆), S-(CH₂)ₙ-phényle, SO-alkyle(C₁-C₆), SO-(CH₂)ₙ-phényle, SO₂-alkyle(C₁-C₆), SO₂-(CH₂)ₙ-phényle, n pouvant aller de 0 à 6 et le radical phényle pouvant être jusqu'à deux fois substitué par F, Cl, Br, OH, CF₃, NO₂, CN, OCF₃, O-alkyle(C₁-C₆), alkyle en C₁-C₆, NH₂ ;
NH₂, NH-alkyle(C₁-C₆), N-[alkyle(C₁-C₆)]₂, NH-acyle(C₁-C₇), phényle, O-(CH₂)ₙ-phényle, n pouvant aller de 0 à 6, le cycle phényle pouvant être une à trois fois substitué par F, Cl, Br, I, OH, CF₃, NO₂, CN, OCF₃, O-alkyle(C₁-C₆), alkyle en C₁-C₆, NH₂, NH-alkyle(C₁-C₆), N[alkyle(C₁-C₆)]₂, SO₂CH₃, COOH, COO-alkyle (C₁-C₆), CONH₂ ;
L représente 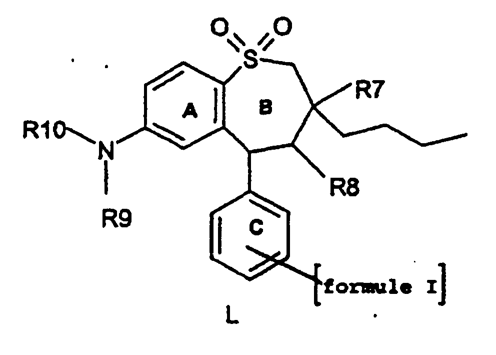
R7 représente le groupe méthyle, éthyle, propyle, butyle ;
R8 représente H, OH, NH₂, un groupe NH-alkyle(C₁-C₆) ;
R9 représente le groupe méthyle, éthyle, propyle, butyle ;
R10 représente le groupe méthyle, éthyle, propyle, butyle ;
l'un des radicaux R1 et R3 ayant la signification d'un groupement -CH₂)₀₋₁-NH-(C=O)₀₋₁-alkylène(C₃-C₂₅)-(C=O)₀₋₁-NH-L, un ou plusieurs atomes de carbone du radical alkylène pouvant être remplacés par des atomes d'oxygène ;
ainsi que leurs sels pharmaceutiquement acceptables.

5. Médicament contenant un ou plusieurs des composés selon une ou plusieurs des revendications 1 à 4.

6. Médicament contenant un ou plusieurs des composés selon une ou plusieurs des revendications 1 à 4 et au moins une autre substance active.

7. Médicament selon la revendication 6, **caractérisé en ce qu'**il contient comme autre substance active un ou plusieurs composés qui régularisent le métabolisme des lipides.

8. Médicament selon la revendication 6, **caractérisé en ce qu'**il contient comme autre substance active un(e) ou plusieurs antidiabétiques, principes actifs hypoglycémiants, inhibiteurs de HMGCoA-réductase, inhibiteurs de la résorption du cholestérol, agonistes de PPAR gamma, agonistes de PPAR alpha, agonistes de PPAR alpha/gamma, fibrates, inhibiteurs de MTP, inhibiteurs de résorption d'acides biliaires, inhibiteurs de CTEP, adsorbeurs polymères d'acides biliaires, inducteurs de récepteurs de LDL, inhibiteurs d'ACAT, antioxydants, inhibiteurs de lipoprotéine lipase, inhibiteurs d'ATP-citrate lyase, inhibiteurs de squalène synthétase, antagonistes de lipoprotéine (a), inhibiteurs de lipase, insulines, sulfonylurées, biguanides, méglitinides, thiazolidinediones, inhibiteurs d'α-glucosidase, principes actifs agissant sur le canal potassique ATP-dépendant des cellules bêta, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, agonistes de MSH (hormone simulant les mélanocytes), agonistes de CCK, inhibiteurs de la recapture de sérotonine, composés sérotoniner-giques et noradrénergiques mixtes, agonistes de 5HT, agonistes de bombésine, antagonistes de galanine, hormones de croissance, composés libérant l'hormone de croissance, agonistes de TRH, modulateurs de protéine 2 ou 3 découpleurs, agonistes de leptine, agonistes de DA (bromocriptine, doprexine), inhibiteurs de lipase/amylase, modulateurs de PPAR, modulateurs de RXR ou agonistes de TR-β ou amphétamines.

9. Composés selon une ou plusieurs des revendications 1 à 4, pour utilisation en tant que médicament destiné au traitement de troubles du métabolisme des lipides.

10. Procédé pour la fabrication d'un médicament contenant un ou plusieurs des composés selon une ou plusieurs des revendications 1 à 4, **caractérisé en ce qu'**on mélange la substance active avec un véhicule pharmaceutiquement convenable et on met ce mélange sous une forme appropriée à l'administration.

11. Utilisation des composés selon une ou plusieurs des revendications 1 à 4, pour la fabrication d'un médicament destiné au traitement de l'hyperlipidémie.

12. Utilisation des composés selon une ou plusieurs des revendications 1 à 4, pour la fabrication d'un médicament destiné à abaisser le taux de cholestérol sérique.

13. Utilisation des composés selon une ou plusieurs des revendications 1 à 4, pour la fabrication d'un médicament destiné au traitement de manifestations artérioscléreuses.

14. Utilisation des composés selon une ou plusieurs des revendications 1 à 4, pour la fabrication d'un médicament destiné au traitement de la résistance à l'insuline.
